(19) Europäisches Patentamt
European Patent Office
Office européen des brevets

(11) **EP 3 403 091 B1**

(12) **EUROPEAN PATENT SPECIFICATION**

(45) Date of publication and mention of the grant of the patent:
**22.12.2021 Bulletin 2021/51**

(21) Application number: **17738287.6**

(22) Date of filing: **11.01.2017**

(51) Int Cl.:
*G01N 33/50* (2006.01)          *G01N 33/53* (2006.01)
*C12Q 1/68* (2018.01)

(86) International application number:
**PCT/IL2017/050037**

(87) International publication number:
**WO 2017/122203 (20.07.2017 Gazette 2017/29)**

(54) **METHODS OF DETERMINING PROGNOSIS OF SEPSIS AND TREATING SAME**

VERFAHREN ZUR BESTIMMUNG DER PROGNOSE VON SEPSIS UND BEHANDLUNG DAVON

MÉTHODES DE DÉTERMINATION DU PRONOSTIC DE SEPTICÉMIE ET TRAITEMENT ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.01.2016 US 201662277016 P**

(43) Date of publication of application:
**21.11.2018 Bulletin 2018/47**

(73) Proprietor: **Technion Research & Development Foundation Limited**
**Technion City, Haifa 3200004 (IL)**

(72) Inventors:
 • **SHEN-ORR, Shai S.**
  **1292600 Karkur (IL)**
 • **ALPERT, Ayelet**
  **3200003 Haifa (IL)**
 • **STAROSVETSKY, Elina**
  **2069363 Yokneam Illit (IL)**
 • **MANSUR, Ashham**
  **37085 Göttingen (DE)**

(74) Representative: **Dennemeyer & Associates S.A.**
**Postfach 70 04 25**
**81304 München (DE)**

(56) References cited:
 • **RAQUEL ALMANSA ET AL: "Transcriptomic correlates of organ failure extent in sepsis",** JOURNAL OF INFECTION., vol. 70, no. 5, 31 December 2014 (2014-12-31), pages 445-456, XP055654678, GB ISSN: 0163-4453, DOI: 10.1016/j.jinf.2014.12.010

 • **MONSERRAT JORGE ET AL: "Clinical relevance of the severe abnormalities of the T cell compartment in septic shock patients",** CRITICAL CARE, BIOMED CENTRAL LTD., LONDON, GB, vol. 13, no. 1, 25 February 2009 (2009-02-25), page R26, XP021053498, ISSN: 1364-8535, DOI: 10.1186/CC7731

 • **CHARALAMBOS A. GOGOS ET AL: "Pro- versus Anti-inflammatory Cytokine Profile in Patients with Severe Sepsis: A Marker for Prognosis and Future Therapeutic Options",** JOURNAL OF INFECTIOUS DISEASES. JID, vol. 181, no. 1, 1 January 2000 (2000-01-01), pages 176-180, XP055654682, US ISSN: 0022-1899, DOI: 10.1086/315214

 • **Jennifer P. Chou ET AL: "T Cell Replicative Senescence in Human Aging",** Current Pharmaceutical Design, 1 March 2013 (2013-03-01), pages 1680-1698, XP055654686, United Arab Emirates DOI: 10.2174/138161213805219711 Retrieved from the Internet: URL:https://www.ncbi.nlm.nih.gov/pmc/articles/PMC3749774/pdf/nihms497521.pdf

 • **RAUL DE PABLO ET AL: "Role of Circulating Lymphocytes in Patients with Sepsis",** BIOMED RESEARCH INTERNATIONAL, vol. 2014, 1 January 2014 (2014-01-01), pages 1-11, XP055654689, ISSN: 2314-6133, DOI: 10.1155/2014/671087

- G. K. PHILIPS ET AL: "Therapeutic uses of anti-PD-1 and anti-PD-L1 antibodies", INTERNATIONAL IMMUNOLOGY, vol. 27, no. 1, 16 October 2014 (2014-10-16), pages 39-46, XP055217958, ISSN: 0953-8178, DOI: 10.1093/intimm/dxu095
- INOUE, SHIGEAKI ET AL.: 'Persistent inflammation and T cell exhaustion in severe sepsis in the elderly' CRITICAL CARE, [Online] vol. 18, no. 3, 24 June 2014, page R130, XP021195402 Retrieved from the Internet: <URL:https://ccforum.biomedcentral.com/articles/10.1186/cc13941>
- SOLANA, RAFAEL ET AL.: 'CMV and Immunosenescence: from basics to clinics' IMMUNITY & AGEING, [Online] vol. 9, no. 1, 31 October 2012, page 23, XP021142257 Retrieved from the Internet: <URL:https://immunityageing.biomedcentral.com/articles/10.1186/1742-4933-9-23>
- CHANG, KATHERINE ET AL.: 'Targeting the programmed cell death 1: programmed cell death ligand 1 pathway reverses T cell exhaustion in patients with sepsis' CRITICAL CARE, [Online] vol. 18, no. 1, 04 January 2014, page R3, XP021175280 Retrieved from the Internet: <URL:https://ccforum.biomedcentral.com/articles/10.1186/cc 13176>
- MARSHALL JOHN C.: "Why have clinical trials in sepsis failed?", TRENDS IN MOLECULAR MEDICINE, vol. 20, no. 4, 1 April 2014 (2014-04-01), pages 195-203, XP55815771, GB ISSN: 1471-4914, DOI: 10.1016/j.molmed.2014.01.007

Remarks:
The complete document including Reference Tables and the Sequence Listing can be downloaded from the EPO website

**Description**

The present invention, relates to an ex-vivo method of determining prognosis of a subject diagnosed with sepsis.

Sepsis is a severe life-threatening systemic inflammatory response related to infection that is responsible for more than 750,000 deaths annually in the US. It is a systemic inflammatory response syndrome (SIRS) with proven or probable infection of bacterial, fungal or viral origin. Severe sepsis is characterized by additional existence of organ dysfunction, while septic shock is defined as sepsis together with the failure of the cardiovascular system to sustain adequate tissue perfusion (Kojic, D, et al., 2015. "Are there new approaches for diagnosis, therapy guidance and outcome prediction of sepsis?". World J. Exp. Med. 5(2): 50-63).

The clinical evaluation of the sepsis condition includes general symptoms such as aberrances of body temperature, fluid balance, glucose metabolism or mental confusion, as well as laboratory indications of inflammation or signs of hemodynamic impairment and organ dysfunction. Marshall JC, et. al. 1995 (Crit Care Med. 1995 Oct;23(10):1638-52) provides a detailed "Multiple organ dysfunction score" (MODS) for determining sepsis condition of a patient.

The immune response in sepsis is composed of two opposing components, namely immune activation, which includes a state of persistent inflammation [Gentile LF, et al., 2012 "Persistent inflammation and immunosuppression: a common syndrome and new horizon for surgical intensive care". J. Trauma Acute Care Surg. 72(6):1491-501] followed by immune-suppression, which includes impaired immune cell effector function [Boomer JS, et al., 2011. "Immunosuppression in patients who die of sepsis and multiple organ failure". JAMA, 306(23):2594-605]. One major contribution to immune-suppression in sepsis is related to T-cell exhaustion, a less-functional state of T-cells induced by long-lasting activation. Besides, other changes in the immune compartment occur in sepsis and relate to cell types' abundances, expression of inhibitory receptors and signaling responses. The resulting sepsis-induced immune suppression is characterized by a collapse of cellular immune response and an increased risk for opportunistic infections with high mortality.

The incidence of sepsis is disproportionately increased in elderly adults, and age is an independent predictor of mortality in sepsis. Compared with younger sepsis patients, elderly nonsurvivors of sepsis die earlier during hospitalization and elderly survivors more frequently require skilled nursing or rehabilitative care after hospitalization.

T-cell exhaustion represents a wide range of dysfunctional states of cytotoxic T-cells arising as a result of chronic inflammation. Historically, the term "exhausted T-cells" referred to terminally differentiated effector memory CD8+T cells that accumulate in older adults who experience chronic inflammation in a process known as: immunesenescence. As terminally differentiated cells who lack proliferative capability, these cells often referred to as "senescent cells". Such senescent CD8+ T cells are highly effector, secreting mainly IPN$\gamma$ and TNF$\alpha$, therefore contributing to the hyperinflammatory state in older adults known as: "inflammaging". The major hallmark of this cell subset is the lack of expression of the co-stimulatory molecule CD28, and increased expression of the replicative senescent marker, CD57.

In human aging, senescent CD8 T cells have been shown to frequently bear antigen specificity against cytomegalovirus (CMV), suggesting that this common and persistent infection may drive immune senescence and result in functional and phenotypic changes in the T cell repertoire. Senescent T cells have also been identified in patients with certain cancers, autoimmune diseases and chronic infections, such as HIV. These T cells differ in phenotype, function and survival from the exhausted T cells that develop in response to highly replicating viruses.

Additional background art: Hotchkiss et al. 2013 Nature Reviews Immunology 13(12): 862/874;

Hotchkiss et al. 2011 The Lancet Infectious Diseases 13(3), 260-268;

Inoue S, et al., 2014 (Persistent inflammation and T-cell exhaustion in severe sepsis in the elderly. Crit Care. 18(3):R130);

Chang K, et al., 2014 (Targeting the programmed cell death 1: programmed cell death ligand 1 pathway reverses T-cell exhaustion in patients with sepsis. Crit Care. 2014 Jan 4;18(1):R3. doi: 10.1186/cc13176);

U.S. Patent Application No. 20150284459;

U.S. Patent Application No. 20150274835;

Rohit Mittal, et al. ("Getting older can be exhausting", Crit Care. 2014; 18(4): 465);

U.S. Patent Application No. 20140199334;

Pottayil G. N. et al., Crit Care. 2011;15(2):R77);

U.S. Patent Application Publication No. 20120021414;

Brice Gaudillière et al. 2014. Sci Transl Med 6, 255ra131.

RAQUEL ALMANSA ET AL, relates to "Transcriptomic correlates of organ failure extent in sepsis", JOURNAL OF INFECTION., GB, (20141231), vol. 70, no. 5, doi:10.1016/j.jinf.2014.12.010, ISSN 0163-4453, pages 445 - 456.

MONSERRAT JORGE ET AL, relates to "Clinical relevance of the severe abnormalities of the T cell compartment in septic shock patients", CRITICAL CARE, BIOMED CENTRAL LTD., LONDON, GB, (20090225), vol. 13, no. 1, doi:10.1186/CC7731, page R26.

CHARALAMBOS A. GOGOS ET AL, relates to "Pro- versus Anti-inflammatory Cytokine Profile in Patients with Severe Sepsis: A Marker for Prognosis and Future Therapeutic Options", JOURNAL OF INFECTIOUS DISEASES.

JID, US, (20000101), vol. 181, no. 1.

Jennifer P. Chou ET AL, relates to "T Cell Replicative Senescence in Human Aging", Current Pharmaceutical Design, United Arab Emirates, doi:10.2174/138161213805219711, (20130301), pages 1680 - 1698, URL: https://www.ncbi.nlm.nih.gov/pmc/articles/PMC3749774/pdf/nihms497521.pdf

RAUL DE PABLO ET AL, relates to "Role of Circulating Lymphocytes in Patients with Sepsis", BIOMED RESEARCH INTERNATIONAL, (20140101), vol. 2014, doi:10.1155/2014/671087, ISSN 2314-6133, pages 1 - 11.

G. K. PHILIPS ET AL, relates to "Therapeutic uses of anti-PD-1 and anti-PD-L1 antibodies", INTERNATIONAL IMMUNOLOGY, (20141016), vol. 27, no. 1, doi:10.1093/intimm/dxu095, ISSN 0953-8178, pages 39 - 46.

## SUMMARY OF THE INVENTION

The present invention relates to an ex-vivo method of determining prognosis of a subject diagnosed with sepsis, comprising determining a baseline level of T-cell senescence in a CD3+ T-cell sample of the subject up to 24 hours following diagnosis of said sepsis, as determined by at least two positive SIRS criteria, said level being indicative of the sepsis prognosis, wherein said T cell-senescence level is determined by the signature $TIM3_{low}$; $CD45RA_{high}$; $CD45RO_{low}$; $CD152_{low}$; $PD1_{low}$, thereby determining the prognosis of the subject wherein if the T-cell senescence level is higher than a predetermined threshold then the subject is likely to have a poor prognosis as compared to a control subject diagnosed with sepsis and whose T-cell senescence level is lower than a predetermined threshold.

Preferably, said T-cell senescence is determined in CD8 and/or CD4 T-cells.

Preferably, it comprises determining in a blood sample of the subject presence of CMV active or latent infection, wherein said presence of said CMV infection indicates that the subject is likely to have a poor prognosis as compared to a control subject diagnosed with sepsis and who is not infected with CMV.

Preferably, said determining is performed using an RNA detection method or a protein detection method.

## BRIEF DESCRIPTION OF THE SEVERAL VIEWS OF THE DRAWINGS

In the drawings:

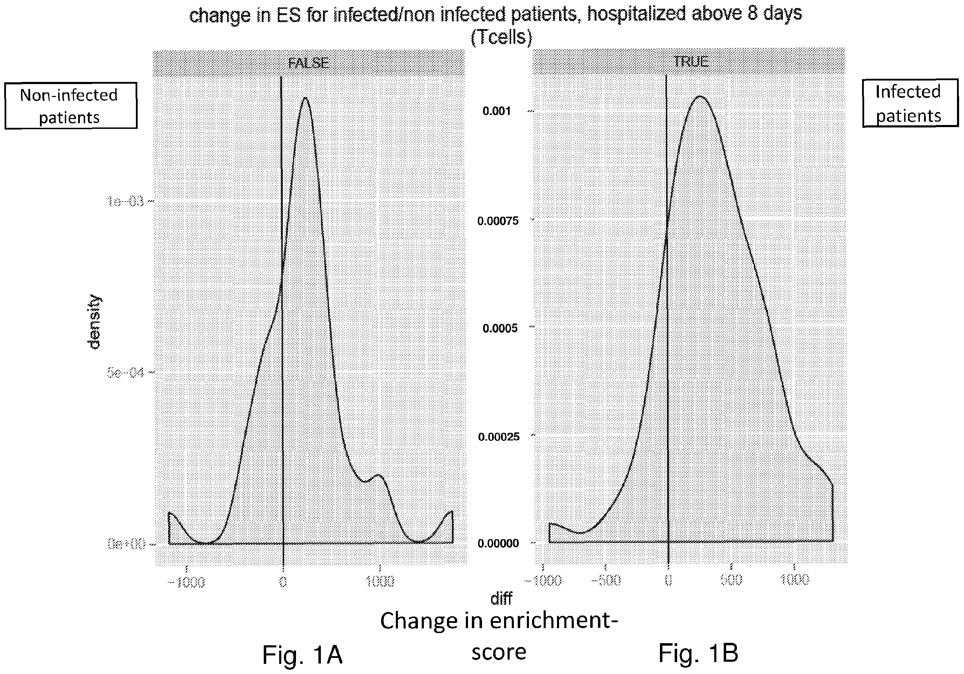

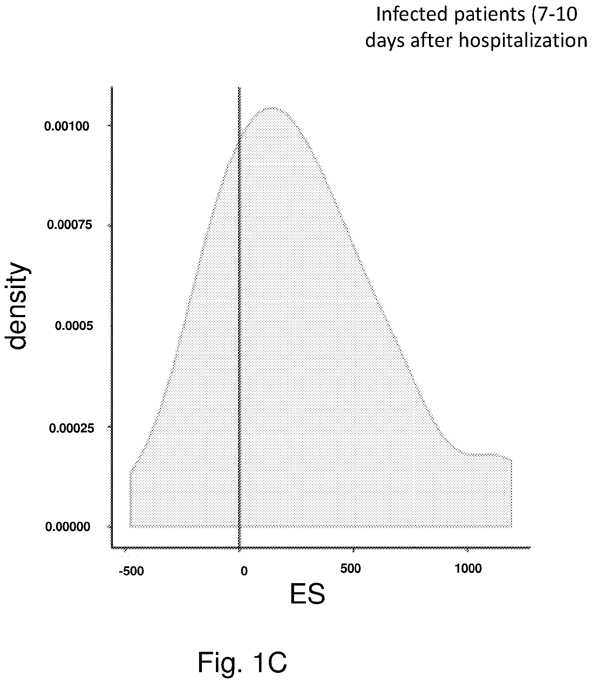

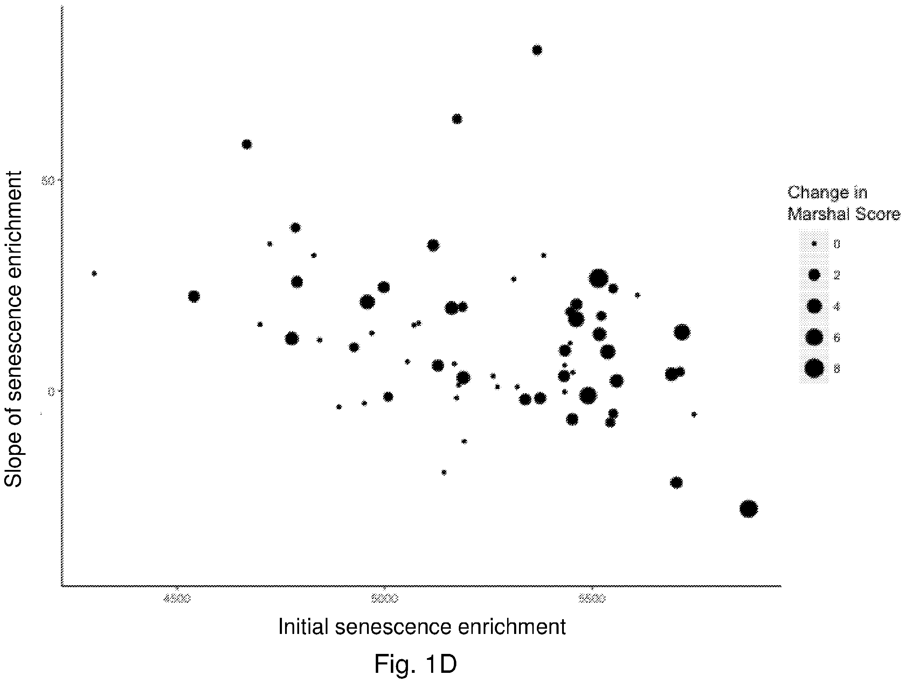

FIGs. 1A-D depict changes in enrichment score (ES) of trauma patients with time. Figures 1A-C - Density histograms describing the change in T-cell senescence enrichment score during hospitalization of trauma patients which were infected (Figures 1B and 1C) or non-infected (Figure 1A). Figures 1A-B - Each value on the "X" axis represents the enrichment score as measured at the later time point (following 8 days of hospitalization) relative to the value of enrichment score measured less than 24 hours since admission to the ICU. Positive values correspond to increase in enrichment and negative to decrease in enrichment. Figure 1C - Each value of the "X" axis represents the enrichment score as measured at the later time point (following 7-10 days of hospitalization) relative to the value of enrichment score measured less than 24 hours since admission to the ICU. Positive values correspond to increase in enrichment and negative to decrease in enrichment. Note that during hospitalization there is a significant increase in T-cell senescence enrichment score among both infected and non-infected patients (p value < 0.01). Figure 1D - Effect of initial and change in senescence enrichment-score on the change of Marshal severity score (MODS) during hospitalization. Each circle represents a single patient. The size of each circle represents the change in MODS score during hospitalization as compared to its value in the initial day of hospitalization. Thus, larger circles represent worsening of the sepsis outcome (higher increase in MODS score). In contrast to the change in senescence, initial enrichment-score significantly affects change in Marshal-score during hospitalization (p value $10e^{-8}$). These results demonstrate that T-cell senescence is strongly induced in sepsis and is related to outcome.

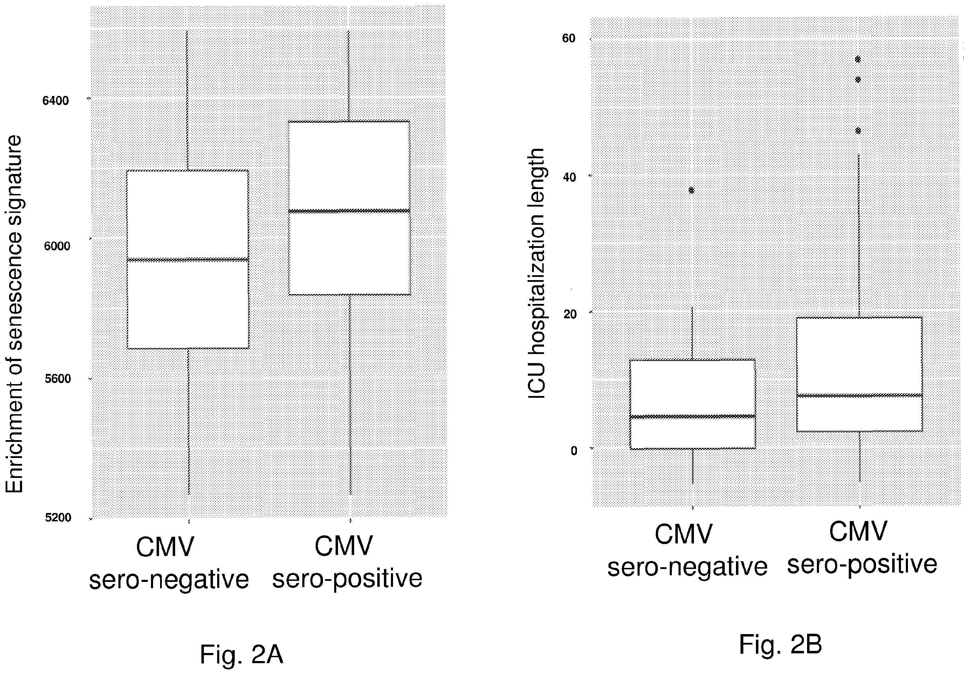

FIGs. 2A-B depict enrichment of T-cell senescence signature (Figure 2A) and ICU hospitalization length (Figure 2B) as depended on CMV serology status. Figure 2A - The present inventors calculated the enrichment of genetic senescence signature in the Framingham gene-expression data-set of old individuals. Note that CMV serology is significantly related to enrichment of senescence signature (p value < $10^{-15}$). Figure 2B - ICU hospitalization length of CMV sero-positive and CMV sero-negative septic patients. CMV sero-positive patients exhibit significantly prolonged hospitalization period as compared to CMV sero-negative patients (p value < 0.02). These results demonstrate that CMV is correlated with high enrichment of genomic senescence signature and with ICU length of stay of septic patients.

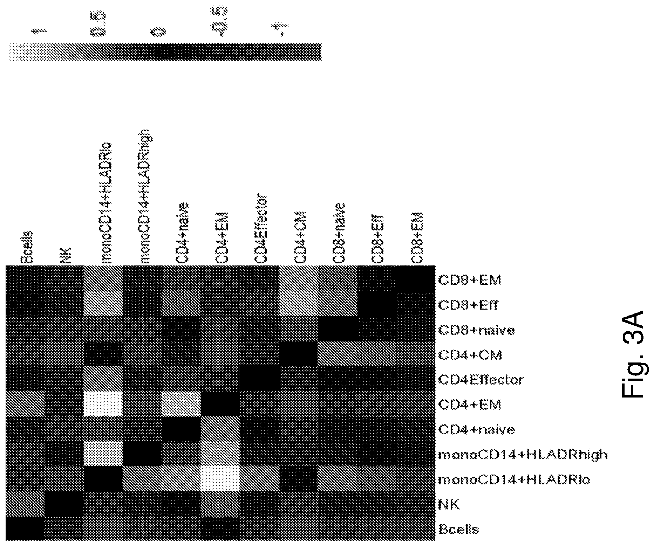

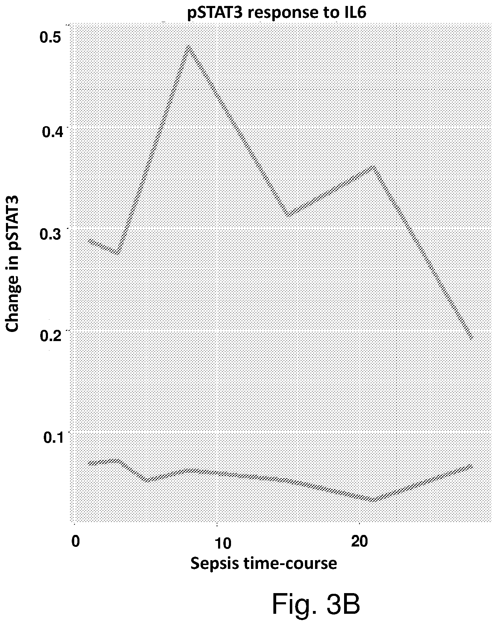

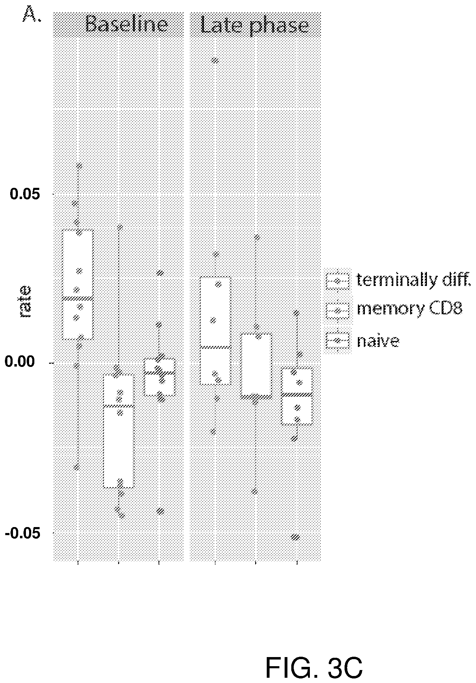

FIGs. 3A-C depict abundances of cell subsets change with sepsis time-course. PBMCs from a septic patient were analyzed in 7 time-points during sepsis time-course (days: 1, 3, 5, 8, 14, 21, and 28). Figure 3A - Log ratio between abundances of different cell types at day 1 (under the diagonal) and day 28 (above the diagonal). The asymmetry of the matrix indicates that the ratio between abundances different cell types differs between day 1 and day 28 in sepsis time course. Figure 3B - pSTAT3 response to IL6 of senescent and non-senescent CD8+T-cells populations with time shows that there is an attenuated response of senescent cells (pink) to IL6-stimulation compared to non- senescent cells (blue). Figure 3C - Increased proportion of senescent cells during sepsis. Calculation of the change in T-cell substypes' abundances at 2 time-points during sepsis: early (5-10 days following sepsis) and late (10-15 days following sepsis) compared to baseline (sepsis diagnosis). During the early phase, the change in abundance of terminally differentiated cells is significantly greater than zero while the change in abundance of memory CD8+Tcells is significantly less than 0. In the late phase of sepsis, changes in abundances of both declined compared to the early phase.

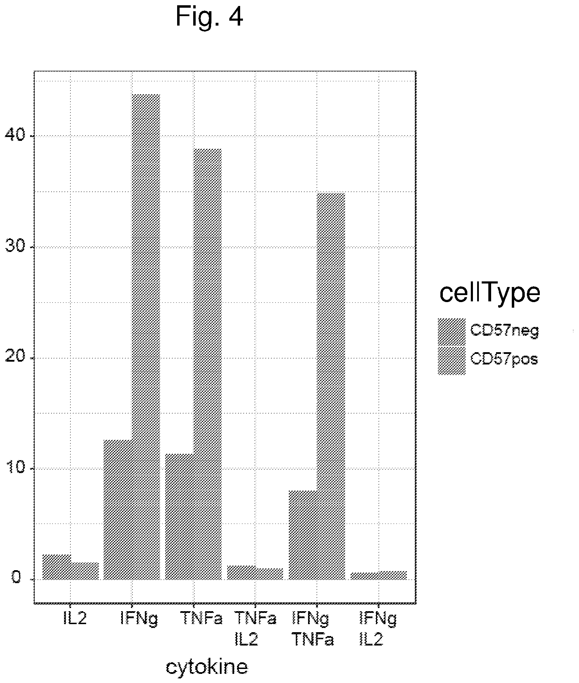

FIG. 4 shows that the CD57positive cell population exhibits a proinflammatory secretion profile. The proportion of cells from each population secreting one or combination of two cytokines is shown (pink for CD57negative CD8+T cells, and blue for CD57positive CD8+T cells).

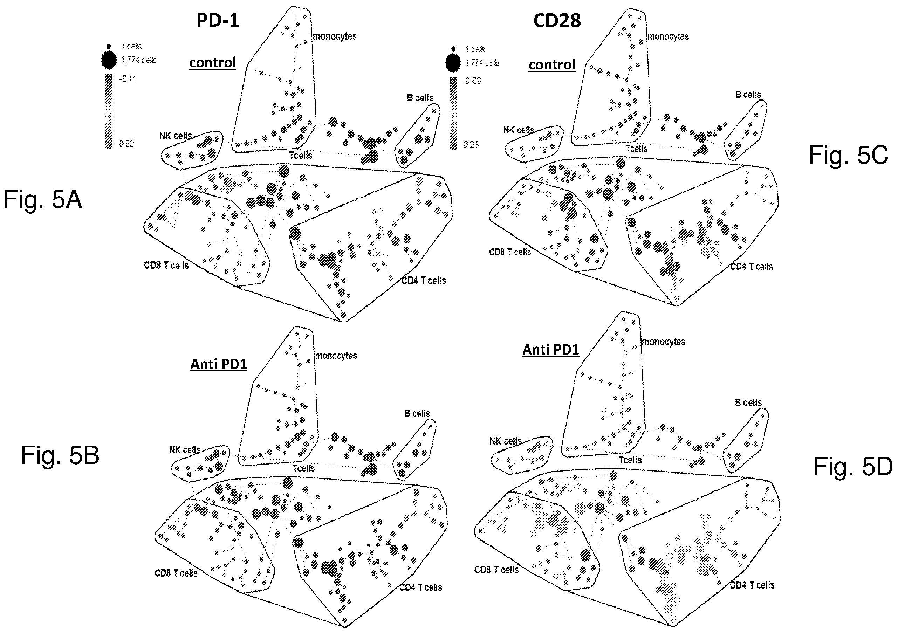

FIGs. 5A-D show that anti-PD-1-treatment restores effector functions in T-cells. PBMCs from a septic patient were extracted 15 days following the onset of sepsis, and were divided into a control group (Figures 5A and 5C) and a group which was treated with PD-1-blocking antibodies (Nivolumab) for 18 hours (Figure 5B and 5D). The present inventors characterized different cell populations by CyTOF using 34 different extra-cellular markers. SPADE is an unsupervised hierarchical clustering method which enables visualization of different cell populations (based on expression of cell-type specific extra-cellular markers) and their relative expression of functional markers. Each circle represents a cluster of similar cells, and its color represents the median value of a specific marker's expression. Figures 5A-B - Expression of PD-1 was lower in the anti PD-1 treated cells (Figure 5B) as compared to the control groups (Figure 5A). Figures 5C-D - Expression of the co-stimulatory molecule was higher in the anti PD-1 treated cells (Figure 5D) as compared to the control group (Figure 5C);

## DESCRIPTION OF SPECIFIC EMBODIMENTS OF THE INVENTION

The present invention is a method as defined by independent claim 1.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The present inventors uncovered a significant increase in the enrichment of T-cell senescence -signature during sepsis (Figures 1A-C, Table 9; Example 1 of the Examples section which follows). Baseline senescence enrichment was significantly correlated with severity of sepsis outcome (according to the MODS Marshal scoring). Moreover, the present inventors found that CMV sero-positive patients were hospitalized in the ICU for significantly prolonged time-periods as compared to CMV sero-negative patients, establishing the clinical impact of CMV serology on outcome in sepsis (Figure 2B, Example 3 of the Examples section which follows). In addition, the present inventors observed that senescent CD8+ T-cell subsets responded poorly to IL6-stimulation reflecting a functional defect of senescent T-cells in sepsis (Figure 3B, Example 4 of the Examples section which follows). The present inventors further showed the CD57positive cell population exhibits a proinflammatory secretion profile (Figure 4, Example 5 of the Examples section which follows).

Moreover, the present inventors demonstrate a high-resolution profiling of septic peripheral blood samples that have been treated *in-vitro* with anti-PD-1-antibodies, which revealed a down-regulation of PD-1 by T-cells, suggesting anti PD1 as a potential treatment for sepsis by inducing the expression of CD28 (Figures 5A-D, Example 6 of the Examples section which follows). These results suggest that anti-PD-1-treatment can be used to reinvigorate the immune-response in sepsis.

According to an aspect of some embodiments of the invention there is provided a method of determining prognosis of a subject diagnosed with sepsis, comprising determining a level of T-cell senescence in the subject, the level being indicative of the sepsis prognosis, thereby determining the prognosis of the subject.

The phrases "sepsis" refers to a life-threatening medical condition which involves "systemic inflammatory response syndrome (SIRS)" with proven or probable infection of bacterial, fungal or viral origin. According to some embodiments of the invention, the sepsis encompasses severe sepsis, septic shock and/or Multiple Organ Dysfunction Syndrome. Severe sepsis is characterized by additional existence of organ dysfunction, while septic shock is defined as sepsis together with the failure of the cardiovascular system to sustain adequate tissue perfusion [Dellinger RP, et al., 2013. "Surviving sepsis campaign: international guidelines for management of severe sepsis and septic shock: 2012". Crit Care Med. 2013 Feb;41(2):580-637; Kojic, D, et al., 2015. "Are there new approaches for diagnosis, therapy guidance and outcome prediction of sepsis?". World J. Exp. Med. 5(2): 50-63.

The diagnosis of SIRS criteria can be made if at least two of the SIRS criteria described are met (marked as "yes" in the below Table 1, sepsis onset).

*Table 1, SIRS criteria*

Table 1: "Temp" = temperature;

| SIRS Criteria (≥ 2 meets SIRS definition) | |
|---|---|
| Temp > 38°C (100.4°F) or < 36°C (96.8°F) | YES / NO |
| Heart Rate > 90 | YES / NO |
| Respiratory Rate > 20 or PaCO2 < 32 mm Hg | YES / NO |
| WBC > 12,000/mm>3, < 4,000/mm>3, or > 10% bands | YES / NO |

The diagnosis of sepsis can be made when SIRS is diagnosed and the source of infection is suspected or present, as described in Table 2, below. Severe sepsis can be diagnosed as described in Table 3, and septic shock and multiple organ dysfunction syndrome can be diagnosed according to Tables 4 and 5, respectively.

**Table 2, sepsis criteria**

Table 2.

| Sepsis Criteria (SIRS + Source of Infection) | |
|---|---|
| Suspected or Present Source of Infection | YES / NO |

**Table 3, severe sepsis criteria**

Table 3.

| Severe Sepsis Criteria (Organ Dysfunction, Hypotension, or Hypoperfusion) | |
|---|---|
| Lactic Acidosis, SBP <90 or SBP Drop ≥ 40 mm Hg of normal | YES / NO |

**Table 4, septic shock criteria**

Table 4.

| septic shock criteria | |
|---|---|
| Severe Sepsis with Hypotension, despite adequate fluid resuscitation | YES / NO |

**Table 5, Multiple Organ Dysfunction Syndrome Criteria**

Table 5.

| Multiple Organ Dysfunction Syndrome Criteria | |
|---|---|
| Evidence of ≥ 2 Organs Failing | YES / NO |

Sepsis prognosis can be defined based on Marshal's multiple organ dysfunction score (MODS score), as described in Marshall JC, Cook DJ, Christou NV, et. al. 1995 ("Multiple organ dysfunction score: a reliable descriptor of a complex clinical outcome". Crit Care Med. 1995 Oct;23(10):1638-52. Review).

Following is a non-limiting description of the criteria used for determining the sepsis state.

*Table 6*

Table 6.

| p aO2/FiO2 Ratio | |
|---|---|
| | 300-1000 (0 points) |
| | 226-300 (1 point) |
| | 151-225 (2 points) |
| | 76-150 (3 points) |
| | 0-75 (4 points) |
| **Platelet Count ($10^3$/mm$^3$)** | |
| | > 120 (0 points) |
| | 81-120 (1 point) |
| | 51-80 (2 points) |
| | 21-50 (3 points) |
| | <= 20 (4 points) |
| **Serum Bilirubin** | |
| | <= 1.2 mg/dL or <= 20 mmol/L (0 points) |
| | 1.2-3.5.....21-60 (1 point) |
| | 3.5-7.0 ..... 61-120 (2 points) |
| | 7.0-14.... 121-240 (3 points) |
| | > 14.....> 240 (4 points) |
| **Pressure Adjusted Heart Rate (HR*CVP/MAP)** | |
| | 0-10 (0 points) |
| | 10.1-15 (1 point) |
| | 15.1-20 (2 points) |
| | 20.1-30 (3 points) |
| | 30.1-300 (4 points) |
| **Glasgow Coma Scale** | |
| | 15-15 (0 points) |
| | 13-14 (1 point) |
| | 10-12 (2 points) |
| | 7-9 (3 points) |
| | 0-6 (4 points) |
| **Serum Creatinine** | |
| | <= 1.1 mg/dL or <= 100 mmol/L (0 points) |
| | 1.1-2.3.....101-200 (1 point) |
| | 2.3-4.0.....201-350 (2 points) |
| | 4.0-5.7 ..... 351-500 (3 points) |
| | > 5.7 ......> 500 (4 points) |

After assigning the suitable score (point per criteria) the total criteria point is counted to reveal the MODS score:

*Table 7*

*MODS Score*

Table 7. ICU = intensive care unit. "Hosp" = hospital; "Mort" = mortality.

| MODS Score | Description of sepsis outcome |
|---|---|
| 0 points: | ICU Mort 0%, Hosp Mort 0%, ICU Stay 2 Days |
| 1-4 points: | ICU Mort 1-2%, Hosp Mort 7%, ICU Stay 3 Days |
| 5-8 points: | ICU Mort 3-5%, Hosp Mort 16%, ICU Stay 6 Days |
| 9-12 points: | ICU Mort 25%, Hosp Mort 50%, ICU Stay 10 Days |
| 13-16 points: | ICU Mort 50%, Hosp Mort 70%, ICU Stay 17 Days |
| 17-20 points: | ICU Mort 75%, Hosp Mort 82%, ICU Stay 21 Days |
| 21-24 points: | ICU Mort 100%, Hospital Mortality 100% |

The term "senescence" refers to terminally differentiated effector memory cells as a state of T-cell dysfunction that arises from repetitive stimulation of CD8+T cells occuring during many viral infections, particularly in the context of aging. T-cell senescence is distinguished from exhaustion in that it arises not through chronic stimulation of the TCR, but from repetitive stimulation. It is defined by increased secretion of pro-inflammatory cytokines such as: IFN$\gamma$ and TNF$\alpha$, lack of IL2 secretion, poor proliferative capacity, and a transcriptional state distinct from that of functional memory T-cells. T-cell senescence may result from both extrinsic negative regulatory pathways (e.g., cytokines) as well as intrinsic strong activation of the TCR.

Multiple markers have been shown to be uniquely up or downregulated on the surface of senescent T-cells. These include upregulation of CD57 and downregulation of CD27, CD28 and CD127. Key transcription factors such as tBET are further strongly expressed in senescent CD8+T cells.

Of note T-cell senescence is not to be interchanged with T-cell exhaustion.

Thus senescent T cells can be identified by a phenotypic marker (e.g., cell proliferation); functional marker (e.g., inflammatory activity); gene expression at the RNA or protein levels for T-cell senescence markers or signatures; and a combination of same. The invention relates to a specific signature of 5 markers.

The terms "high" and "low" throughout the document refers to relative to the respective level in exhausted cells while being determined in the same assay e.g., CyTOF or FACS.

The term "subject" as used herein refers to a mammal, preferably a human being (male or female) at any age.

According to a specific embodiment, the subject is an adult human being.

According to a specific embodiment, the subject is 60 years or older.

According to some embodiments of the invention, the subject is diagnosed with sepsis.

As used herein the phrase "determining a level of T-cell senescence in the subject" refers to determining the signature specified in independent claim 1.

The level of T-cell senescence is determined in a CD3+ T-cell sample of the subject.

According to some embodiments of the invention, the T-cell senescence is determined in CD8 and/or CD4 T-cells.

According to some embodiments of the invention, the T-cell senescence is determined in CD8 T-cells.

As used herein, the phrase "level of expression" refers to the degree of gene expression and/or gene product level or activity in a specific cell. For example, up-regulation or down-regulation of various genes can affect the level of the gene product (i.e., RNA and/or protein) in a specific cell. It should be noted that the gene product can also be referred to as a marker (usually a protein marker, but can also include an RNA marker). Additionally or alternatively, upregulation of a non-coding gene (e.g., on the RNA level) such as a specific pseudogene can be also referred to as a senescence marker.

Methods of determining the level of expression include RNA and/or protein detection methods which are well-known in the art. Non-limiting examples of such methods are further described herein below.

According to some embodiments of the invention, determining the level of expression is performed using an RNA detection method.

According to some embodiments of the invention, determining the level of expression is performed using a protein detection method (e.g., FACS or CyTOF).

According to some embodiments of the invention, determining the level of expression is performed by enumerating (e.g., counting) senescent T-cells.

According to some embodiments of the invention, determining pro-inflammatory activity.

Any of the above represents a separate embodiment that can be combined.

According to some embodiments of the invention, determining the level of expression is performed within a predetermined time period after diagnosing the sepsis.

According to a specific embodiment, determining senescence is determining baseline level of T cell senescence.

As used herein "baseline level of T cell senescence" refers to not more than 24 hours from sepsis onset or up to 24 hours following diagnosis of sepsis (which is typically followed by immediate hospitalization for a non-hospitalized patient).

The present inventors have shown that increased baseline levels of T cell senescence (as compared to control) are indicative of the prognosis.

According to some embodiments of the invention, the predetermined time period comprises up to 2 hours following a diagnosis of the sepsis.

According to some embodiments of the invention, the predetermined time period comprises up to 6 hours following a diagnosis of the sepsis. According to some embodiments of the invention, the predetermined time period comprises up to 12 hours following a diagnosis of the sepsis.

According to some embodiments of the invention, the predetermined time period comprises up to 24 hours following a diagnosis of the sepsis.

According to some embodiments of the invention, determining the level of T-cell senescence is performed in a septic patient having a MODS score higher than 0 points.

According to a specific embodiment, determining the level of T-cell senescence is performed up to 5 days following sepsis onset.

Thus, according to the method of some embodiments of the invention, the level of the T-cell senescence is indicative of the sepsis prognosis.

According to some embodiments of the invention, wherein when the level of a senescence marker is higher than a predetermined threshold then the subject is likely to have a poor prognosis as compared to the average value of a cohort of subjects diagnosed with sepsis characterized by levels of expression of the T cell senescence marker or combination of T-cell senescence markers that are lower than a predetermined threshold (negative control).

Alternatively the control may be a positive control e.g., of subjects having poor prognosis and exhibiting high level of T cell senescence such as the cohort described herein in the Examples section which follows.

A poor prognosis refers to hospitalization length and increase in MODS score during hospitalization. The latter is associated with poor survival. In such cases, the patient is treated intensively according to the present symptoms broad range-antibiotic, supportive treatment, intensive care unit and anti-T cell exhaustion therapy.

According to some embodiments of the invention, the control subject recovered from the sepsis following about one, two, or three weeks.

According to some embodiments of the invention, in the control subject the expression of the at least one T-cell senescence marker is lower than a predetermined threshold.

As used herein the phrase "predetermined threshold" refers to at least about 5%, e.g., at least about 10 %, e.g., higher than about 20 %, e.g., higher than about 30 %, e.g., higher than about 40 %, e.g., higher than about 50 %, e.g., higher than about 60 %, higher than about 70 %, higher than about 80 %, higher than about 90 %, higher than about 2 times, higher than about three times, higher than about four time, higher than about five times, higher than about six times, higher than about seven times, higher than about eight times, higher than about nine times, higher than about 20 times, higher than about 50 times, higher than about 100 times, higher than about 200 times, higher than about 350, higher than about 500 times, higher than about 1000 times, or more relative to the reference expression data.

As used herein "higher" or "lower" refers to statistically significant values.

As used herein the phrase "reference expression data" refers to the expression level of the at least one gene in a healthy subject of a similar age, and/or with respect to the level of expression of at least one gene which expression level thereof is not changed (e.g., not increased) during the course of sepsis (e.g., a gene which is not changed during T-cell senescence).

According to some embodiments of the invention, wherein when the level of expression of the at least one T-cell senescence marker or combination is higher than a predetermined threshold then the subject is likely to have a poor prognosis characterized by an MODS score of 5 or higher and equivalent scores (e.g. APACHE II/III and SOFA scores).

Sequence information regarding genes (e.g., genomic sequences, e.g., in case of pseudogenes) and the gene products (*i.e.*, RNA transcripts and polypeptide sequences) of the T-cell senescence marker(s), which are upregulated in sepsis, can be found in Table 9 herein below (Example 1 of the Examples section which follows).

According to some aspects of the disclosure, the T-cell senescence marker is the polynucleotide set forth by SEQ ID NOs: 1-406, 814-888, and 946-949.

According to some aspects of the disclosure, the T-cell senescence marker is the polynucleotide set forth by SEQ ID NOs: 1-406, 814-888, and 946-949.

According to some aspects of the disclosure, the T-cell senescence marker is the polypeptide set forth by SEQ ID NOs: 407-813 and 889-945.

According to some aspects of the disclosure, the T-cell senescence marker is the polypeptide set forth by SEQ ID NOs: 407-813 and 889-945, and 946-949.

It will be appreciated that marker data can be used to assess the frequency of the population of senescent T-cells and hence prognose the sepsis.

It should be noted that probes suitable for detecting the T-cell senescence marker(s) can be designed by those skilled in the art based on the sequences of the DNA and RNA encoding the marker(s). Similarly, antibodies which can specifically bind to the protein markers can be synthesized and/or isolated by those skilled in the art, and/or can be purchased from commercial suppliers of antibodies.

Once antibodies are obtained, they may be tested for activity, for example via ELISA, Western blot, FACS, immunohistochemical analysis and/or RIA as is further described hereinunder.

As shown in Figure 2A, CMV seropositive was found to be highly associated with enrichments of senescence signature, as well as with length of ICU hospitalization. Thus, the present inventors have envisaged that CMV-seropositive can predict the prognosis of sepsis.

According to some embodiments of the invention, the method further comprising determining in a blood sample of the subject presence of CMV serum positive (CMV infection), wherein the presence of the CMV serum positive (CMV infection) indicates that the subject is likely to have a poor prognosis as compared to a control subject diagnosed with sepsis and who is not infected with CMV.

According to some embodiments of the invention, the method further comprising determining in a blood sample of the subject presence of antibodies to CMV and/or detection of CMV RNA (e.g., by real time PCR or any other method), wherein the presence of the antibodies to CMV and/or the CMV RNA indicates that the subject is likely to have a poor prognosis as compared to a control subject diagnosed with sepsis and who is not infected with CMV.

Classically, detection of IgG antibodies to CMV is diagnostic of past infection and synonymous with the term "seropositivity".

Serum-positivity can be detected by any number of assays for antigen or DNA from actively replicating virus. For example, detection of the early matrix protein pp65, as occurs with a positive CMV "rapid antigen" test or detection of CMV DNA by means of polymerase chain reaction (PCR) from peripheral blood leukocytes or bodily fluid or biopsy material, is an indication of actively replicating virus.

CMV serum positive can be detected using methods well known in the art.

Following is a non-limiting description of an assay which can text CMV pp65 antigenemia: pp65 antigenemia can be determined in blood collected in EDTA tubes and subjected to dextran sedimentation (1% dextran in phosphate-buffered saline). Duplicates of $5 \times 10^5$ leukocytes are placed onto glass slides, and the pp65 antigen-positive cells are evaluated by immunofluorescence assay (IFA) by using a mixture of 2 monoclonal mouse anti-pp65 antibodies (20:1; Virion, Rüschlikon, Switzerland; Argene Biosoft, Viva Diagnostika, Hürth, Germany) and goat anti-mouse immunoglobulin (Ig) G (Dianova, Hamburg, Germany) conjugated with fluorescein isothiocyanate (FITC).

Following is a non-limiting description of an assay movitors presence of CMV. CMV monitoring can include virus culture (human foreskin fibroblast monolayers) from tracheal secretions, qualitative nested PCR targeting the CMV IE1-Ex4 region from leukocytes, plasma and tracheal secretions, and quantification of CMV-DNA (COBAS Amplicor CMV Monitor™ test, Roche Diagnostics, Mannheim, Germany) from qualitative PCR-positive plasma and tracheal secretion specimens.

CMV serology can be done by detecting anti-CMV IgG and anti-CMV IgM using enzyme immunoassays (e.g., from Medac, Wedel, Germany; e.g., enzyme-linked immunosorbent assay (DiaSorin, Düsseldorf, Germany)).

A status of viral latency is assigned if anti-CMV immunoglobulin G (IgG) is present but the virus cannot be detected otherwise. Since earlier investigations had shown that healthy seropositive blood donors deliver negative CMV PCR results from leukocytes and plasma, CMV-DNA detection in plasma, leukocytes or respiratory secretions or positive virus isolation is defined as CMV-reactivation, which is clinically indicative for an immune suppressed state.

As is shown in Figure 1D and as is further described in the Examples section which follows, the baseline levels of T-cells senescence were highly correlated with a worsened sepsis prognosis as was shown by the significant increase in MODS (Marchal score).

There is provided a method of monitoring a subject diagnosed with sepsis, the method comprising determining at least twice during the course of the sepsis a level of T-cell senescence in the subject, thereby monitoring a subject diagnosed with sepsis.

The level of T cell senescence is a baseline level and constitutes at least one determination when a plurality of determinations are performed.

According to an aspect of some embodiments of the present invention the method uses a composition-of-matter comprising serum of a sepsis patient and a reagent which specifically binds a T-cell senescence marker (e.g., as described herein).

According to some embodiments of the invention, the reagent comprises an antibody capable of specifically binding the T-cell senescence marker.

According to some embodiments of the invention, the composition of-matter further comprises a secondary antibody capable of specifically binding the antibody capable of specifically binding the T-cell senescence marker.

According to some embodiments of the invention, the antibody is conjugated to a detectable moiety.

Various types of detectable or reporter moieties may be conjugated to the antibody of the invention. These include, but not are limited to, a radioactive isotope (such as [125]iodine), a phosphorescent chemical, a chemiluminescent chemical, a fluorescent chemical (fluorophore), an enzyme, a fluorescent polypeptide, an affinity tag, and molecules (contrast agents) detectable by Positron Emission Tomography (PET) or Magnetic Resonance Imaging (MRI).

Examples of suitable fluorophores include, but are not limited to, phycoerythrin (PE), fluorescein isothiocyanate (FITC), Cy-chrome, rhodamine, green fluorescent protein (GFP), blue fluorescent protein (BFP), Texas red, PE-Cy5, and the like. For additional guidance regarding fluorophore selection, methods of linking fluorophores to various types of molecules see Richard P. Haugland, "Molecular Probes: Handbook of Fluorescent Probes and Research Chemicals 1992-1994", 5th ed., Molecular Probes, Inc. (1994); U.S. Pat. No. 6,037,137 to Oncoimmunin Inc.; Hermanson, "Bioconjugate Techniques", Academic Press New York, N.Y. (1995); Kay M. et al., 1995. Biochemistry 34:293; Stubbs et al., 1996. Biochemistry 35:937; Gakamsky D. et al., "Evaluating Receptor Stoichiometry by Fluorescence Resonance Energy Transfer," in "Receptors: A Practical Approach," 2nd ed., Stanford C. and Horton R. (eds.), Oxford University Press, UK. (2001); U.S. Pat. No. 6,350,466 to Targesome, Inc.]. Fluorescence detection methods which can be used to detect the antibody when conjugated to a fluorescent detectable moiety include, for example, fluorescence activated flow cytometry (FACS), immunofluorescence confocal microscopy, fluorescence *in-situ* hybridization (FISH) and fluorescence resonance energy transfer (FRET).

Numerous types of enzymes may be attached to the antibody of the invention [e.g., horseradish peroxidase (HPR), beta-galactosidase, and alkaline phosphatase (AP)] and detection of enzyme-conjugated antibodies can be performed using ELISA (e.g., in solution), enzyme-linked immunohistochemical assay (e.g., in a fixed tissue), enzyme-linked chemiluminescence assay (e.g., in an electrophoretically separated protein mixture) or other methods known in the art [see e.g., Khatkhatay MI. and Desai M., 1999. J Immunoassay 20:151-83; Wisdom GB., 1994. Methods Mol Biol. 32:433-40; Ishikawa E. et al., 1983. J Immunoassay 4:209-327; Oellerich M., 1980. J Clin Chem Clin Biochem. 18:197-208; Schuurs AH. and van Weemen BK., 1980. J Immunoassay 1:229-49).

The affinity tag (or a member of a binding pair) can be an antigen identifiable by a corresponding antibody [e.g., digoxigenin (DIG) which is identified by an anti-DIG antibody) or a molecule having a high affinity towards the tag [e.g., streptavidin and biotin]. The antibody or the molecule which binds the affinity tag can be fluorescently labeled or conjugated to enzyme as described above.

Various methods, widely practiced in the art, may be employed to attach a streptavidin or biotin molecule to the antibody of the invention. For example, a biotin molecule may be attached to the antibody of the invention via the recognition sequence of a biotin protein ligase (e.g., BirA) as described in the Examples section which follows and in Denkberg, G. et al., 2000. Eur. J. Immunol. 30:3522-3532. Alternatively, a streptavidin molecule may be attached to an antibody fragment, such as a single chain Fv, essentially as described in Cloutier SM. et al., 2000. Molecular Immunology 37:1067-1077; Dubel S. et al., 1995. J Immunol Methods 178:201; Huston JS. et al., 1991. Methods in Enzymology 203:46; Kipriyanov SM. et al., 1995. Hum Antibodies Hybridomas 6:93; Kipriyanov SM. et al., 1996. Protein Engineering 9:203; Pearce LA. et al., 1997. Biochem Molec Biol Intl 42:1179-1188).

Functional moieties, such as fluorophores, conjugated to streptavidin are commercially available from essentially all major suppliers of immunofluorescence flow cytometry reagents (for example, Pharmingen or Becton-Dickinson).

Following is a non-limiting list of identifiable moieties which can be used according to some embodiments of the invention: Green Fluorescent protein, Alkaline phosphatase, Peroxidase, Histidine tag, Myc tag, Biotin lygase tag, orange fluorescent protein, Beta galactosidase, and Streptavidin.

According to some embodiments of the invention, the reagent comprises a polynucleotide capable of specifically hybridizing to and/or extending the nucleotide sequence encoding a specific T-cell senescence marker.

According to some embodiments of the invention, the polynucleotide is labeled (also referred to as a "probe").

Methods of labeling polynucleotides are known in the art, for example, using a radioactive isotope, a non-radioactive label such as Digoxygenin, fluorescent label, biotin (e.g., which can be further detected by a labeled streptavidin) or any identifiable or detectable moiety.

As is shown in Figures 4A-D and described in the Examples section which follows, the present inventors have uncovered that treatment of exhausted T-cells from a septic patient with anti-PD-1-antibodies resulted in down-regulation of PD-1 by T-cells as well as significant induction of CD28, a co-stimulatory receptor essential for T-cells activation. These results suggest that anti-PD-1-treatment might be useful to reinvigorate the immune-response in sepsis.

According to an aspect of some embodiments of the invention patients may be selected by the method of the invention for treatment. It is always to be understood that the invention and its embodiments do not extend to the treatment itself, merely the selection of the patients for treatment.

According to some embodiments of the invention, the subject is selected by determining at least twice during the course of the sepsis or at least at baseline the levels of T-cell senescence in the subject, wherein if the subject exhibits elevated levels of T cell senescence then the subject is qualified for intensive medical treatment e.g., treatment with the anti-T-cell exhaustion therapy.

The term "treating" refers to inhibiting, preventing or arresting the development of a pathology (disease, disorder or condition) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

As used herein the phrase "treatment regimen" refers to a treatment plan that specifies the type of treatment, dosage, schedule and/or duration of a treatment provided to a subject in need thereof (e.g., a subject diagnosed with a pathology). The selected treatment regimen can be an aggressive one which is expected to result in the best clinical outcome (e.g., complete cure of the pathology) or a more moderate one which may relief symptoms of the pathology yet results in incomplete cure of the pathology. It will be appreciated that in certain cases the more aggressive treatment regimen may be associated with some discomfort to the subject or adverse side effects (e.g., damage to healthy cells or tissue). The type of treatment can include a surgical intervention (e.g., removal of lesion, diseased cells, tissue, or organ), a cell replacement therapy, an administration of a therapeutic drug (e.g., receptor agonists, antagonists, hormones, chemotherapy agents) in a local or a systemic mode, an exposure to radiation therapy using an external source (e.g., external beam) and/or an internal source (e.g., brachytherapy) and/or any combination thereof. The dosage, schedule and duration of treatment can vary, depending on the severity of pathology and the selected type of treatment, and those of skills in the art are capable of adjusting the type of treatment with the dosage, schedule and duration of treatment.

It should be noted that the elevation in the level of T-cell senescence may occur between at least two determination steps (2 time periods of determining T-cell senescence during the course of sepsis).

As used herein the phrase "anti-T-cell exhaustion therapy" - refers to an agent which can reverse and/or inhibit the T-cell exhaustion in the subject.

According to some embodiment of the invention, the anti-T-cell exhaustion therapy comprises an immune checkpoint inhibitor.

As used herein the term "immune-check point protein" refers to an antigen independent protein that modulates an immune cell response (i.e. activation or function). Immune-check point proteins can be either co-stimulatory proteins [i.e. positively regulating an immune cell activation or function by transmitting a co-stimulatory secondary signal resulting in activation of an immune cell] or inhibitory proteins (i.e. negatively regulating an immune cell activation or function by transmitting an inhibitory signal resulting in suppressing activity of an immune cell).

According to specific embodiments, the immune-check point protein regulates activation or function of a T cell.

Non-limiting examples of immune-check point proteins include PDL-1 [gene symbol CD274, is also known as CD274 and B7-H1; GenBank accession Numbers NP_054862 and NP_054862], TIM-3 [gene symbol HAVCR2, is also known as Hepatitis A Virus Cellular Receptor 2, T Cell Immunoglobulin Mucin 3, T-Cell Immunoglobulin And Mucin Domain-Containing Protein, T-Cell Membrane Protein 3 and KIM-3; GenBank Number NP_116171], and CD27 [gene symbol CD27, is also known as Tumor Necrosis Factor Receptor Superfamily, Member 7, TNFRSF7 and S152; GenBank Number NP_001233].

According to specific embodiments, the immune checkpoint inhibitor comprises a monoclonal antibody.

Non-limiting examples of suitable immune checkpoint inhibitors include an anti-PD-1 antibody, alemtuzumab, bevacizumab, brentuximab vedotin, cetuximab, gemtuzumab ozogamicin, ibritumomab tiuxetan, ipilimumab (anti-CTLA-4), ofatumumab, panitumumab, rituximab, tositumomab, trastuzumab, anti-B7-H4, anti-B7-H1, anti-LAG3, BTLA, anti-Tim3, anti-B7-DC, anti-CD160, KIR antagonist antibodies, anti-4-1BB, anti-OX40, anti-CD27, and CD40 agonist antibodies.

According to some embodiments of the invention, the anti-T-cell exhaustion therapy comprises an anti-PD-1-antibody.

PD-1 (Programmed Death 1) [gene symbol PDCD1, is also known as CD279; GenBank Number NP_005009] is an example of an immune-check point protein.

Anti-PD-1-antibodies, inhibitors and/or antagonists which are suitable for treating T-cell exhaustion are known in the art. See e.g., U.S. Patent Application Publication No. 20140044738 to Langermann Solomon ("Antibodies And Other Molecules That Bind B7-H1 And PD-1"); U.S. Patent Application Publication No. 20100055102 to Langermann Solomon ("COMPOSITIONS OF PD-1 ANTAGONISTS AND METHODS OF USE"); U.S. Patent Application Publication No. 20120114648 Langermann Solomon et al. ("COMPOSITIONS OF PD-1 ANTAGONISTS AND METHODS OF USE"); U.S. Patent Application Publication No. 20120114649 to Langermann Solomon et al. ("COMPOSITIONS OF PD-1 ANTAGONISTS AND METHODS OF USE"); U.S. Patent Application Publication No. 20100151492 to Ahmed Rafi et al. ("METHODS FOR THE TREATMENT OF INFECTIONS AND TUMORS").

A non-limiting example of an anti-PD-1-antibody which can be used by the method of some embodiments of the invention is KEYTRUDA® (pembrolizumab), Merck's Anti-PD-1 Therapy.

According to some embodiments of the invention, the anti-T-cell exhaustion therapy comprises an anti-IL-27 antibody or inhibitor.

Anti-T-cell exhaustion therapy can include anti-IL-27 inhibitors as described in U.S. Patent Application Publication No. 20150284459 to Kuchroo; Vijay K et al. ("METHODS FOR MODULATING IMMUNE RESPONSES DURING CHRONIC IMMUNE CONDITIONS BY TARGETING IL-27 INDUCED PATHWAYS");

The anti-T-cell exhaustion therapy of some embodiments of the invention can be administered to an organism per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the anti-T-cell exhaustion therapy accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

The term "tissue" refers to part of an organism consisting of cells designed to perform a function or functions. Examples include, but are not limited to, brain tissue, retina, skin tissue, hepatic tissue, pancreatic tissue, bone, cartilage, connective tissue, blood tissue, muscle tissue, cardiac tissue brain tissue, vascular tissue, renal tissue, pulmonary tissue, gonadal tissue, hematopoietic tissue.

Pharmaceutical compositions may be manufactured by processes well known in the art, e.g., by means of conventional mixing, dissolving, granulating, dragee-making, levigating, emulsifying, encapsulating, entrapping or lyophilizing processes.

Pharmaceutical compositions may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition may also be formulated in rectal compositions such as suppositories or retention enemas, using, e.g., conventional suppository bases such as cocoa butter or other glycerides.

Pharmaceutical compositions suitable for use in context of some embodiments of the invention include compositions wherein the active ingredients are contained in an amount effective to achieve the intended purpose. More specifically, a therapeutically effective amount means an amount of active ingredients (anti-T-cell exhaustion therapy) effective to prevent, alleviate or ameliorate symptoms of a pathology (e.g., sepsis) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide tissue levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

### Methods of detecting the expression level of protein

Non-limiting examples of protein detection methods include, flow cytometry (e.g., intra or extra-cellular flow cytometry), FACS, ELISA, Western Blot, RIA, immunohistochemistry, protein activity assays and Mass cytometry (e.g., CyTOF (FLUIDIGM$^R$)).

Mass cytometry uniquely combines time-of-flight mass spectrometry with Maxpar metal-labeling technology to enable breakthrough discovery and comprehensive functional profiling applications. Cellular targets are labeled with metal-tagged antibodies and detected and quantified by time-of-flight mass spectrometry. The high purity and choice of metal isotopes ensure minimal background noise from signal overlap or endogenous cellular components.

*Enzyme linked immunosorbent assay (ELISA):* This method involves fixation of a sample (e.g., fixed cells or a proteinaceous solution) containing a protein substrate to a surface such as a well of a microtiter plate. A substrate specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

*Western blot:* This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies. Antibody binding reagents may be radiolabeled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

*Radio-immunoassay (RIA):* In one version, this method involves precipitation of the desired protein (*i.e.*, the substrate) with a specific antibody and radiolabeled antibody binding protein (e.g., protein A labeled with I$^{125}$) immobilized on a precipitable carrier such as agarose beads. The number of counts in the precipitated pellet is proportional to the amount of substrate.

In an alternate version of the RIA, a labeled substrate and an unlabeled antibody binding protein are employed. A sample containing an unknown amount of substrate is added in varying amounts. The decrease in precipitated counts from the labeled substrate is proportional to the amount of substrate in the added sample.

*Fluorescence activated cell sorting (FACS):* This method involves detection of a substrate *in situ* in cells by substrate specific antibodies. The substrate specific antibodies are linked to fluorophores. Detection is by means of a cell sorting machine which reads the wavelength of light emitted from each cell as it passes through a light beam. This method may employ two or more antibodies simultaneously.

*Immunohistochemical analysis:* This method involves detection of a substrate *in situ* in fixed cells by substrate specific antibodies. The substrate specific antibodies may be enzyme linked or linked to fluorophores. Detection is by microscopy and subjective or automatic evaluation. If enzyme linked antibodies are employed, a colorimetric reaction may be required. It will be appreciated that immunohistochemistry is often followed by counterstaining of the cell nuclei using for example Hematoxyline or Giemsa stain.

*In situ activity assay:* According to this method, a chromogenic substrate is applied on the cells containing an active enzyme and the enzyme catalyzes a reaction in which the substrate is decomposed to produce a chromogenic product visible by a light or a fluorescent microscope.

*In vitro activity assays:* In these methods the activity of a particular enzyme is measured in a protein mixture extracted from the cells. The activity can be measured in a spectrophotometer well using colorimetric methods or can be measured in a non-denaturing acrylamide gel (*i.e.*, activity gel). Following electrophoresis the gel is soaked in a solution containing a substrate and colorimetric reagents. The resulting stained band corresponds to the enzymatic activity of the protein of interest. If well calibrated and within the linear range of response, the amount of enzyme present in the sample is proportional to the amount of color produced. An enzyme standard is generally employed to improve quantitative accuracy.

## Methods of detecting the expression level of RNA

The expression level of the RNA in the cells of some embodiments of the invention can be determined using methods known in the arts.

*Northern Blot analysis:* This method involves the detection of a particular RNA in a mixture of RNAs. An RNA sample is denatured by treatment with an agent (e.g., formaldehyde) that prevents hydrogen bonding between base pairs, ensuring that all the RNA molecules have an unfolded, linear conformation. The individual RNA molecules are then separated according to size by gel electrophoresis and transferred to a nitrocellulose or a nylon-based membrane to which the denatured RNAs adhere. The membrane is then exposed to labeled DNA probes. Probes may be labeled using radioisotopes or enzyme linked nucleotides. Detection may be using autoradiography, colorimetric reaction or chemiluminescence. This method allows both quantitation of an amount of particular RNA molecules and determination of its identity by a relative position on the membrane which is indicative of a migration distance in the gel during electrophoresis.

*RT-PCR analysis:* This method uses PCR amplification of relatively rare RNAs molecules. First, RNA molecules are purified from the cells and converted into complementary DNA (cDNA) using a reverse transcriptase enzyme (such as an MMLV-RT) and primers such as, oligo dT, random hexamers or gene specific primers. Then by applying gene specific primers and Taq DNA polymerase, a PCR amplification reaction is carried out in a PCR machine. Those of skills in the art are capable of selecting the length and sequence of the gene specific primers and the PCR conditions (*i.e.*, annealing temperatures, number of cycles and the like) which are suitable for detecting specific RNA molecules. It will be appreciated that a semi-quantitative RT-PCR reaction can be employed by adjusting the number of PCR cycles and comparing the amplification product to known controls.

*RNA in situ hybridization stain:* In this method DNA or RNA probes are attached to the RNA molecules present in the cells. Generally, the cells are first fixed to microscopic slides to preserve the cellular structure and to prevent the RNA molecules from being degraded and then are subjected to hybridization buffer containing the labeled probe. The hybridization buffer includes reagents such as formamide and salts (e.g., sodium chloride and sodium citrate) which enable specific hybridization of the DNA or RNA probes with their target mRNA molecules *in situ* while avoiding non-specific binding of probe. Those of skills in the art are capable of adjusting the hybridization conditions (*i.e.*, temperature, concentration of salts and formamide and the like) to specific probes and types of cells. Following hybridization, any unbound probe is washed off and the bound probe is detected using known methods. For example, if a radio-labeled probe is used, then the slide is subjected to a photographic emulsion which reveals signals generated using radio-labeled probes; if the probe was labeled with an enzyme then the enzyme-specific substrate is added for the formation of a colorimetric reaction; if the probe is labeled using a fluorescent label, then the bound probe is revealed using a fluorescent microscope; if the probe is labeled using a tag (e.g., digoxigenin, biotin, and the like) then the bound probe can be detected following interaction with a tag-specific antibody which can be detected using known methods.

*In situ RT-PCR stain:* This method is described in Nuovo GJ, et al. [Intracellular localization of polymerase chain reaction (PCR)-amplified hepatitis C cDNA. Am J Surg Pathol. 1993, 17: 683-90] and Komminoth P, et al. [Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract. 1994, 190: 1017-25]. Briefly, the RT-PCR reaction is performed on fixed cells by incorporating labeled nucleotides to the PCR reaction. The reaction is carried on using a specific *in situ* RT-PCR apparatus such as the laser-capture microdissection PixCell I LCM system available from Arcturus Engineering (Mountainview, CA).

## DNA microarrays/DNA chips:

The expression of thousands of genes may be analyzed simultaneously using DNA microarrays, allowing analysis of the complete transcriptional program of an organism during specific developmental processes or physiological responses. DNA microarrays consist of thousands of individual gene sequences attached to closely packed areas on the surface of a support such as a glass microscope slide. Various methods have been developed for preparing DNA microarrays. In one method, an approximately 1 kilobase segment of the coding region of each gene for analysis is individually PCR amplified. A robotic apparatus is employed to apply each amplified DNA sample to closely spaced zones on the surface of a glass microscope slide, which is subsequently processed by thermal and chemical treatment to bind the DNA sequences to the surface of the support and denature them. Typically, such arrays are about 2 x 2 cm and contain about individual nucleic acids 6000 spots. In a variant of the technique, multiple DNA oligonucleotides, usually 20 nucleotides in length, are synthesized from an initial nucleotide that is covalently bound to the surface of a support, such that tens of thousands of identical oligonucleotides are synthesized in a small square zone on the surface of the support. Multiple oligonucleotide sequences from a single gene are synthesized in neighboring regions of the slide for analysis of expression of that gene. Hence, thousands of genes can be represented on one glass slide. Such arrays of synthetic oligonucleotides may be referred to in the art as "DNA chips", as opposed to "DNA microarrays", as described above [Lodish et al. (eds.). Chapter 7.8: DNA Microarrays: Analyzing Genome-Wide Expression. In: Molecular Cell Biology, 4th ed., W. H. Freeman, New York. (2000)].

*Oligonucleotide microarray* - In this method oligonucleotide probes capable of specifically hybridizing with the polynucleotides of some embodiments of the invention are attached to a solid surface (e.g., a glass wafer). Each oligonucleotide probe is of approximately 20-25 nucleic acids in length. To detect the expression pattern of the polynucleotides of some embodiments of the invention in a specific cell sample (e.g., blood cells), RNA is extracted from the cell sample using methods known in the art (using e.g., a TRIZOL solution, Gibco BRL, USA). Hybridization can take place using either labeled oligonucleotide probes (e.g., 5'-biotinylated probes) or labeled fragments of complementary DNA (cDNA) or RNA (cRNA). Briefly, double stranded cDNA is prepared from the RNA using reverse transcriptase (RT) (e.g., Superscript II RT), DNA ligase and DNA polymerase I, all according to manufacturer's instructions (Invitrogen Life Technologies, Frederick, MD, USA). To prepare labeled cRNA, the double stranded cDNA is subjected to an *in vitro* transcription reaction in the presence of biotinylated nucleotides using e.g., the BioArray High Yield RNA Transcript Labeling Kit (Enzo, Diagnostics, Affymetix Santa Clara CA). For efficient hybridization the labeled cRNA can be fragmented by incubating the RNA in 40 mM Tris Acetate (pH 8.1), 100 mM potassium acetate and 30 mM magnesium acetate for 35 minutes at 94 °C. Following hybridization, the microarray is washed and the hybridization signal is scanned using a confocal laser fluorescence scanner which measures fluorescence intensity emitted by the labeled cRNA bound to the probe arrays.

For example, in the Affymetrix microarray (Affymetrix®, Santa Clara, CA) each gene on the array is represented by a series of different oligonucleotide probes, of which, each probe pair consists of a perfect match oligonucleotide and a mismatch oligonucleotide. While the perfect match probe has a sequence exactly complimentary to the particular gene, thus enabling the measurement of the level of expression of the particular gene, the mismatch probe differs from the perfect match probe by a single base substitution at the center base position. The hybridization signal is scanned using the Agilent scanner, and the Microarray Suite software subtracts the non-specific signal resulting from the mismatch probe from the signal resulting from the perfect match probe.

As used herein, the term "antibody" refers to a substantially intact antibody molecule.

As used herein, the phrase "antibody fragment" refers to a functional fragment of an antibody (such as Fab, F(ab')2, Fv or single domain molecules such as VH and VL) that is capable of binding to an epitope of an antigen.

Suitable Antibody fragments for practicing some embodiments of the invention include a complementarity-determining region (CDR) of an immunoglobulin light chain (referred to herein as "light chain"), a complementarity-determining region of an immunoglobulin heavy chain (referred to herein as "heavy chain"), a variable region of a light chain, a variable region of a heavy chain, a light chain, a heavy chain, an Fd fragment, and antibody fragments comprising essentially whole variable regions of both light and heavy chains such as an Fv, a single chain Fv, an Fab, an Fab', and an F(ab')2.

Functional antibody fragments comprising whole or essentially whole variable regions of both light and heavy chains are defined as follows:

(i) Fv, defined as a genetically engineered fragment consisting of the variable region of the light chain and the variable region of the heavy chain expressed as two chains;
(ii) single chain Fv ("scFv"), a genetically engineered single chain molecule including the variable region of the light chain and the variable region of the heavy chain, linked by a suitable polypeptide linker as a genetically fused single chain molecule.
(iii) Fab, a fragment of an antibody molecule containing a monovalent antigen-binding portion of an antibody molecule which can be obtained by treating whole antibody with the enzyme papain to yield the intact light chain and the Fd fragment of the heavy chain which consists of the variable and CH1 domains thereof;
(iv) Fab', a fragment of an antibody molecule containing a monovalent antigen-binding portion of an antibody molecule which can be obtained by treating whole antibody with the enzyme pepsin, followed by reduction (two Fab' fragments are obtained per antibody molecule);
(v) F(ab')2, a fragment of an antibody molecule containing a monovalent antigen-binding portion of an antibody molecule which can be obtained by treating whole antibody with the enzyme pepsin (i.e., a dimer of Fab' fragments held together by two disulfide bonds); and

(vi) Single domain antibodies are composed of a single VH or VL domains which exhibit sufficient affinity to the antigen.

Methods of generating antibodies (i.e., monoclonal and polyclonal) are well known in the art. Antibodies may be generated via any one of several methods known in the art, which methods can employ induction of in-vivo production of antibody molecules, screening of immunoglobulin libraries (Orlandi D.R. et al., 1989. Proc. Natl. Acad. Sci. U. S. A. 86:3833-3837; Winter G. et al., 1991. Nature 349:293-299) or generation of monoclonal antibody molecules by continuous cell lines in culture. These include, but are not limited to, the hybridoma technique, the human B-cell hybridoma technique, and the Epstein-Barr virus (EBV)-hybridoma technique (Kohler G. et al., 1975. Nature 256:495-497; Kozbor D. et al., 1985. J. Immunol. Methods 81:31-42; Cote RJ. et al., 1983. Proc. Natl. Acad. Sci. U. S. A. 80:2026-2030; Cole SP. et al., 1984. Mol. Cell. Biol. 62:109-120).

In cases where target antigens are too small to elicit an adequate immunogenic response when generating antibodies in-vivo, such antigens (haptens) can be coupled to antigenically neutral carriers such as keyhole limpet hemocyanin (KLH) or serum albumin [e.g., bovine serum albumine (BSA)] carriers (see, for example, US. Pat. Nos. 5,189,178 and 5,239,078]. Coupling a hapten to a carrier can be effected using methods well known in the art. For example, direct coupling to amino groups can be effected and optionally followed by reduction of the imino linkage formed. Alternatively, the carrier can be coupled using condensing agents such as dicyclohexyl carbodiimide or other carbodiimide dehydrating agents. Linker compounds can also be used to effect the coupling; both homobifunctional and heterobifunctional linkers are available from Pierce Chemical Company, Rockford, Ill. The resulting immunogenic complex can then be injected into suitable mammalian subjects such as mice, rabbits, and the like. Suitable protocols involve repeated injection of the immunogen in the presence of adjuvants according to a schedule which boosts production of antibodies in the serum. The titers of the immune serum can readily be measured using immunoassay procedures which are well known in the art.

The antisera obtained can be used directly or monoclonal antibodies may be obtained as described hereinabove.

Antibody fragments can be obtained using methods well known in the art. [(see, for example, Harlow and Lane, "Antibodies: A Laboratory Manual", Cold Spring Harbor Laboratory, New York, (1988)]. For example, antibody fragments according to some embodiments of the invention can be prepared by proteolytic hydrolysis of the antibody or by expression in E. coli or mammalian cells (e.g., Chinese hamster ovary cell culture or other protein expression systems) of DNA encoding the fragment.

Alternatively, antibody fragments can be obtained by pepsin or papain digestion of whole antibodies by conventional methods. As described hereinabove, an (Fab')2 antibody fragments can be produced by enzymatic cleavage of antibodies with pepsin to provide a 5S fragment. This fragment can be further cleaved using a thiol reducing agent, and optionally a blocking group for the sulfhydryl groups resulting from cleavage of disulfide linkages to produce 3.5S Fab' monovalent fragments. Alternatively, enzymatic cleavage using pepsin produces two monovalent Fab' fragments and an Fc fragment directly. Ample guidance for practicing such methods is provided in the literature of the art (for example, refer to: Goldenberg, U.S. Pat. Nos. 4,036,945 and 4,331,647; Porter, RR., 1959. Biochem. J. 73:119-126). Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

As described hereinabove, an Fv is composed of paired heavy chain variable and light chain variable domains. This association may be noncovalent (see, for example, Inbar et al., 1972. Proc. Natl. Acad. Sci. USA. 69:2659-62). Alternatively, as described hereinabove the variable domains can be linked to generate a single chain Fv by an intermolecular disulfide bond, or alternately, such chains may be cross-linked by chemicals such as glutaraldehyde.

Preferably, the Fv is a single chain Fv.

Single chain Fv's are prepared by constructing a structural gene comprising DNA sequences encoding the heavy chain variable and light chain variable domains connected by an oligonucleotide encoding a peptide linker. The structural gene is inserted into an expression vector, which is subsequently introduced into a host cell such as E. coli. The recombinant host cells synthesize a single polypeptide chain with a linker peptide bridging the two variable domains. Ample guidance for producing single chain Fv's is provided in the literature of the art (for example, refer to: Whitlow and Filpula, 1991. Methods 2:97-105; Bird et al., 1988. Science 242:423-426; Pack et al., 1993. Bio/Technology 11:1271-77; and Ladner et al., U.S. Pat. No. 4,946,778).

Isolated complementarity determining region peptides can be obtained by constructing genes encoding the complementarity determining region of an antibody of interest. Such genes may be prepared, for example, by RT-PCR of mRNA of an antibody-producing cell. Ample guidance for practicing such methods is provided in the literature of the art (for example, refer to Larrick and Fry, 1991. Methods 2:106-10).

It will be appreciated that for human therapy or diagnostics, humanized antibodies are preferably used. Humanized forms of non-human (e.g., murine) antibodies are genetically engineered chimeric antibodies or antibody fragments having-preferably minimal-portions derived from non-human antibodies. Humanized antibodies include antibodies in which complementary determining regions of a human antibody (recipient antibody) are replaced by residues from a complementarity determining region of a non-human species (donor antibody) such as mouse, rat or rabbit having the desired functionality. In some instances, Fv framework residues of the human antibody are replaced by corresponding non-human residues. Humanized antibodies may also comprise residues which are found neither in the recipient antibody nor in the imported complementarity determining region or framework sequences. In general, the humanized antibody will comprise substantially all of at least one, and typically two, variable domains, in which all or substantially all of the complementarity determining regions correspond to those of a non-human antibody and all, or substantially all, of the framework regions correspond to those of a relevant human consensus sequence. Humanized antibodies optimally also include at least a portion of an antibody constant region, such as an Fc region, typically derived from a human antibody (see, for example, Jones et al., 1986. Nature 321:522-525; Riechmann et al., 1988. Nature 332:323-329; and Presta, 1992. Curr. Op. Struct. Biol. 2:593-596).

Methods for humanizing non-human antibodies are well known in the art. Generally, a humanized antibody has one or more amino acid residues introduced into it from a source which is non-human. These non-human amino acid residues are often referred to as imported residues which are typically taken from an imported variable domain. Humanization can be essentially performed as described (see, for example: Jones et al., 1986. Nature 321:522-525; Riechmann et al., 1988. Nature 332:323-327; Verhoeyen et al., 1988. Science 239:1534-1536; U.S. Pat. No. 4,816,567) by substituting human complementarity determining regions with corresponding rodent complementarity determining regions. Accordingly, such humanized antibodies are chimeric antibodies, wherein substantially less than an intact human variable domain has been substituted by the corresponding sequence from a non-human species. In practice, humanized antibodies may be typically human antibodies in which some complementarity determining region residues and possibly some framework residues are substituted by residues from analogous sites in rodent antibodies.

Human antibodies can also be produced using various techniques known in the art, including phage display libraries [see, for example, Hoogenboom and Winter, 1991. J. Mol. Biol. 227:381; Marks et al., 1991. J. Mol. Biol. 222:581; Cole et al., "Monoclonal Antibodies and Cancer Therapy", Alan R. Liss, pp. 77 (1985); Boerner et al., 1991. J. Immunol. 147:86-95). Humanized antibodies can also be made by introducing sequences encoding human immunoglobulin loci into transgenic animals, e.g., into mice in which the endogenous immunoglobulin genes have been partially or completely inactivated. Upon antigenic challenge, human antibody production is observed in such animals which closely resembles that seen in humans in all respects, including gene rearrangement, chain assembly, and antibody repertoire. Ample guidance for practicing such an approach is provided in the literature of the art (for example, refer to: U.S. Pat. Nos. 5,545,807, 5,545,806, 5,569,825, 5,625,126, 5,633,425, and 5,661,016; Marks et al., 1992. Bio/Technology 10:779-783; Lonberg et al., 1994. Nature 368:856-859; Morrison, 1994. Nature 368:812-13; Fishwild et al., 1996. Nature Biotechnology 14:845-51; Neuberger, 1996. Nature Biotechnology 14:826; Lonberg and Huszar, 1995. Intern. Rev. Immunol. 13:65-93).

It will be appreciated that targeting of particular compartment within the cell can be achieved using intracellular antibodies (also known as "intrabodies"). These are essentially SCA to which intracellular localization signals have been added (e.g., ER, mitochondrial, nuclear, cytoplasmic). This technology has been successfully applied in the art (for review, see Richardson and Marasco, 1995, TIBTECH vol. 13). Intrabodies have been shown to virtually eliminate the expression of otherwise abundant cell surface receptors and to inhibit a protein function within a cell (See, for example, Richardson et al., 1995, Proc. Natl. Acad. Sci. USA 92: 3137-3141; Deshane et al., 1994, Gene Ther. 1: 332-337; Marasco et al., 1998 Human Gene Ther 9: 1627-42; Shaheen et al., 1996 J. Virol. 70: 3392-400; Werge, T. M. et al., 1990, FEBS Letters 274:193-198; Carlson, J.R 1993 Proc. Natl. Acad. Sci. USA 90:7427-7428; Biocca, S. et al., 1994, Bio/Technology 12: 396-399; Chen, S-Y. et al., 1994, Human Gene Therapy 5:595-601; Duan, L et al., 1994, Proc. Natl. Acad. Sci. USA 91:5075-5079; Chen, S-Y. et al., 1994, Proc. Natl. Acad. Sci. USA 91:5932-5936; Beerli, R.R. et al., 1994, J. Biol. Chem. 269:23931-23936; Mhashilkar, A.M. et al., 1995, EMBO J. 14:1542-1551; PCT Publication No. WO 94/02610 by Marasco et al.; and PCT Publication No. WO 95/03832 by Duan et al.).

To prepare an intracellular antibody expression vector, the cDNA encoding the antibody light and heavy chains specific for the target protein of interest are isolated, typically from a hybridoma that secretes a monoclonal antibody specific for the marker. Hybridomas secreting anti-marker monoclonal antibodies, or recombinant monoclonal antibodies, can be prepared using methods known in the art. Once a monoclonal antibody specific for the marker protein is identified (e.g., either a hybridoma-derived monoclonal antibody or a recombinant antibody from a combinatorial library), DNAs encoding the light and heavy chains of the monoclonal antibody are isolated by standard molecular biology techniques. For hybridoma derived antibodies, light and heavy chain cDNAs can be obtained, for example, by PCR amplification or cDNA library screening. For recombinant antibodies, such as from a phage display library, cDNA encoding the light and heavy chains can be recovered from the display package (e.g., phage) isolated during the library screening process and the nucleotide sequences of antibody light and heavy chain genes are determined. For example, many such sequences are disclosed in Kabat, E. A., et al. (1991) Sequences of Proteins of Immunological Interest, Fifth Edition, U.S. Department of Health and Human Services, NIH Publication No. 91-3242 and in the "Vbase" human germline sequence database. Once obtained, the antibody light and heavy chain sequences are cloned into a recombinant expression vector using standard methods.

For cytoplasmic expression of the light and heavy chains, the nucleotide sequences encoding the hydrophobic leaders of the light and heavy chains are removed. An intracellular antibody expression vector can encode an intracellular antibody in one of several different forms. For example, in one embodiment, the vector encodes full-length antibody light and heavy chains such that a full-length antibody is expressed intracellularly. In another embodiment, the vector encodes a full-length light chain but only the VH/CH1 region of the heavy chain such that a Fab fragment is expressed intracellularly. In another embodiment, the vector encodes a single chain antibody (scFv) wherein the variable regions of the light and heavy chains are linked by a flexible peptide linker [e.g., $(Gly_4Ser)_3$ and expressed as a single chain molecule. To inhibit marker activity in a cell, the expression vector encoding the intracellular antibody is introduced into the cell by standard transfection methods, as discussed hereinbefore.

As used herein the term "about" refers to $\pm$ 10 %

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

## GENERAL MATERIALS AND EXPERIMENTAL METHODS

**ssGSEA (single sample GSEA):** The method was first described by Barbie et al [2009; Barbie, D. A., et al., 2009. Systematic RNA interference reveals that oncogenic KRAS -driven cancers require TBK1, 462(November)] and allows one to define an enrichment-score that represents the degree of absolute enrichment of a gene-set in a given sample. Following ordering the expression of the genes participate in the signature according to their rank, the enrichment score for each sample is calculated by the differences between the ECDF (empirical cumulative distribution functions) of the genes participate in the signature and the genes that are not.

**Samples collection:** The present inventors collected peripheral blood from 67 septic patients longitudinally across their hospitalization period in the intensive care unit (ICU). For each patient, samples were taken roughly at days 1, 7 and 14 following sepsis diagnosis. PBMCs and serums were isolated using Ficoll density gradient. More than 90% of the patients were older than 50 years, thereby more affected by the high-grade inflammation that occurs in sepsis. Since CMV induces T-cell replicative senescence, the present inventors recorded the CMV serology of the patients. As expected, about one quarter of the septic patients were CMV sero-negative while the others were CMV sero-positive.

MODS scoring was based on Marshall JC, Cook DJ, Christou NV, et. al. Multiple organ dysfunction score: a reliable descriptor of a complex clinical outcome. Crit Care Med. 1995 Oct;23(10):1638-52. Review.

Measurements of CMV serology was performed using standard procedures.

**Stimulation tests with IL6:** Peripheral blood samples from the septic patients were incubated with interleukin 6 (IL6) at a concentration of 200 ng/ml (nanogams per milliliter) for 15 minutes (time to reach peak activation). The reaction was stopped by fixation (10 minutes) using paraformaldehide. The cells were stained with the extra-cellular antibodies panel (detailed in Table 3) and then permeabilized in methanol. Afterwards the cells were stained with the intra-cellular panel containing phospho-STATs: pSTAT1, pSTAT3 and pSTAT5.

**Treatment of peripheral blood with anti-PD-1-antibodies:** PBMCs from a septic patient were extracted 15 days following the onset of sepsis, and were treated with PD-1 blocking antibodies (Nivolumab) for 18 hours.

**CyTOF analysis:** CyTOF (Fludigm) is a recently introduced mass-cytometer capable of detecting up to 40 markers conjugated to heavy metals simultaneously on single cells. The present inventors built a panel of 36 markers (provided in Table 8 below) including phenotypic and functional markers. The phenotypic markers enable discrimination between more than 10 different cell types. The functional markers include markers that undergo up- or down-regulation in senescence of T-cells. Each sample was stained with the antibodies in the panel and introduced to CyTOF. Normalization of the signal between the runs was done by means of standard-elements beads. Initial data-analysis was performed by Cytobank.

*Table 8*

*List of markers used in CyTOF analysis*

Table 8.

| Marker name | Expression in senescent T-cells |
|---|---|
| CD8a | High |
| CD95 | Low |
| IL21R | Low |
| BTLA | High |
| CD62L | High |
| CD4 | High |
| CD11c | Low |
| KLRG1 | High |
| CD123 | Low |
| CD7 | Low |
| pSTAT5 | Low |
| CD130 | Low |
| CD27 | High |
| pSTAT1 | Low |
| CD69 | Low |
| CD25 | Low |
| CD19 | Low |
| PDL1 | Low |
| pSTAT3 | Low |
| CD33 | Low |
| Tbet | High |
| CD14 | Low |
| CD126; IL6R | High |
| CD127 | High |
| TIM3 | Low |
| CD45RO | High |
| CD16 | Low |
| CD28 | High |
| CD152 | Low |
| CCR7 | Low |
| CD45RA | High |
| PD-1 | Low |
| CD11b | Low |
| CD3 | High |
| HLA-DR | Low |
| CD57 | High |
| CD45 | High |

*Citrus:* The complexity of discrimination between cell subsets grows exponentially with the number of markers used. For x markers, there are at least $2^x$ combinations of different expression levels. Therefore, the use of automated cell clustering methods becomes essential when analyzing CyTOF data. The present inventors use Citrus, an unsupervised hierarchical clustering algorithm that stratifies cells into clusters based on the distance between their markers' expression.

*Quantification of response to IL6:* As IL6 induces phosphorylation of STAT3, the present inventors calculated the distributions of pSTAT3 levels with or without IL6 stimulation. The response to IL6 was defined as the area between both distributions.

*EXAMPLE 1*

*BASELINE T-CELL SENESCENCE AT SEPSIS PREDICTS PROGNOSIS OF SEPSIS*

*Experimental results*

For assessing the dynamics of T-cell senescence in sepsis, the present inventors performed enrichment analysis of gene-expression data of septic patients. As HIV is a major driving force of senescence, the present inventors utilized gene-expression data of HIV-specific T-cells for construction of an exhaustion genetic-signature composed of genes that undergo up-regulation in senescence T-cells. Using ssGSEA (single-sample GSEA) (Gene-Set enrichment analysis) the present inventors computed the enrichment of the senescence signature at baseline hospitalization and over time in a longitudinal gene expression dataset of trauma patients that contracted nosocomial infections. The present inventors observed a significant increase in the enrichment of senescence - signature during sepsis (**Figures 1A-C**).

Table 9 hereinbelow provides details of the T-cell senescence genetic-signature identified by the present inventors.

*Table 9*

| Gene/Marker | Secreted/not secreted? | Polynucleotide SEQ ID NO: | Polypeptide SEQ ID NO: |
|---|---|---|---|
| A2M | secreted | 1 | 407 |
| ACLY | secreted | 2-3 | 408-409 |
| ACTB | secreted | 4 | 410 |
| ACTR2 | secreted | 5-6 | 411-412 |
| ACTR3 | secreted | 7 | 413 |
| ADGRG 1 | secreted | 833-843 | 911-921 |
| AGBL2 | Not Secreted | 8 | 414 |
| AIM2 | Not Secreted | 9 | 415 |
| ANXA2 | secreted | 10-13 | 416-419 |
| APBB1IP | Not Secreted | 14 | 420 |
| ARG2 | Not Secreted | 15 | 421 |
| ARHGAP25 | Not Secreted | 16-19 | 422-425 |
| ARID5B | Not Secreted | 20 | 426 |
| ARL6IP5 | secreted | 21 | 427 |
| ARPC5 | secreted | 22 | 428 |
| ASUN | Not Secreted | 831 | 909 |
| ATRN | secreted | 23-24 | 429-430 |
| ATXN10 | secreted | 25-26 | 431-432 |
| AZI2 | Not Secreted | 27-29 | 433-435 |
| BACE1 | Not Secreted | 30-33 | 436-439 |
| BARD1 | Not Secreted | 34 | 440 |

(continued)

| Gene/Marker | Secreted/not secreted? | Polynucleotide SEQ ID NO: | Polypeptide SEQ ID NO: |
|---|---|---|---|
| BATF | Not Secreted | 35 | 441 |
| BAZ1A | Not Secreted | 36-37 | 442-443 |
| BLVRA | secreted | 38 | 444 |
| BST2 | secreted | 39 | 445 |
| C18orf25 | Not Secreted | 40-41 | 446-447 |
| C1GALT1 | Not Secreted | 42 | 448 |
| C6orf47 | Not Secreted | 43 | 449 |
| C8orf44 | Not Secreted | 44 | 450 |
| CA5B | Not Secreted | 45 | 451 |
| CALML4 | Not Secreted | 46-47 | 452-453 |
| CASP1 | secreted | 48-52 | 454-458 |
| CASP4 | Not Secreted | 53-54 | 459-460 |
| CASP7 | Not Secreted | 55-58 | 461-464 |
| CBR1 | secreted | 59 | 465 |
| CCL4 | secreted | 60 | 466-467 |
| CCL5 | secreted | 61 | 468 |
| CCR5 | Not Secreted | 62-63 | 469-470 |
| CCT3 | secreted | 64-65 | 471-472 |
| CCT7 | secreted | 66-69 | 473-476 |
| CD244 | Not Secreted | 70-72 | 477-479 |
| CD27 | secreted | 73 | 480 |
| CD38 | secreted | 74 | 481 |
| CD46 | secreted | 75-82 | 482-489 |
| CD52 | Not Secreted | 83 | 490 |
| CDK2 | Not Secreted | 84-85 | 491-492 |
| CDKL1 | secreted | 86 | 493 |
| CENPU | Not Secreted | 829-830 | 907-908 |
| CHI3L2 | secreted | 87-89 | 494-496 |
| CLEC2B | Not Secreted | 90 | 497 |
| CTBP1 | Not Secreted | 91-92 | 498-499 |
| CTSC | secreted | 93-95 | 500-502 |
| CX3CR1 | Not Secreted | 96-99 | 503-506 |
| DBI | secreted | 100-102 | 507-509 |
| DCAF11 | Not Secreted | 103-105 | 510-512 |
| DDX46 | Not Secreted | 106 | 513 |
| DDX60 | Not Secreted | 107 | 514 |
| DENND2D | Not Secreted | 108 | 515 |
| DHCR24 | Not Secreted | 109 | 516 |

(continued)

| Gene/Marker | Secreted/not secreted? | Polynucleotide SEQ ID NO: | Polypeptide SEQ ID NO: |
|---|---|---|---|
| DHFR | Not Secreted | 110 | 517 |
| DHFRP1 | | 877 | #N/A |
| DIAPH1 | Not Secreted | 111-112 | 518-519 |
| DNAJC1 | Not Secreted | 113 | 520 |
| DONSON | Not Secreted | 114 | 521 |
| DZIP3 | Not Secreted | 115 | 522 |
| EIF2S2P4 | | 878 | #N/A |
| ENPP4 | secreted | 116 | 523 |
| EPM2A | Not Secreted | 117-118 | 524-525 |
| EXOC2 | Not Secreted | 119 | 526 |
| FANCL | Not Secreted | 120-121 | 527-528 |
| FDPS | Not Secreted | 122-124 | 529-531 |
| FOXN3-AS2 | | 849 | #N/A |
| FYB | Not Secreted | 125-126 | 532-533 |
| GALK2 | Not Secreted | 127-128 | 534-535 |
| GART | secreted | 129-132 | 536-539 |
| GBP1 | secreted | 133 | 540 |
| GBP1P1 | | 850 | #N/A |
| GBP2 | Not Secreted | 134 | 541 |
| GFOD1 | secreted | 135 | 542 |
| GGCT | secreted | 136 | 543 |
| GPD2 | Not Secreted | 137-138 | 544-545 |
| GTF2I | Not Secreted | 139-143 | 546-550 |
| GYG1 | secreted | 144 | 551 |
| GZMA | secreted | 145 | 552 |
| GZMB | Not Secreted | 146 | 553 |
| GZMH | Not Secreted | 147 | 554 |
| HAL | Not Secreted | 148 | 555 |
| HELLS | Not Secreted | 149 | 556 |
| HEMK1 | Not Secreted | 150 | 557 |
| HERC6 | Not Secreted | 151-152 | 558-559 |
| HIBCH | secreted | 153-154 | 560-561 |
| HIST1H4H | secreted | 155-168 | 562-575 |
| HMGB1P3 | | 879 | #N/A |
| HOXB9 | Not Secreted | 169 | 576 |
| HRSP12 | secreted | 170 | 577 |
| IBTK | Not Secreted | 171 | 578 |
| ICT1 | Not Secreted | 172 | 579 |

(continued)

| Gene/Marker | Secreted/not secreted? | Polynucleotide SEQ ID NO: | Polypeptide SEQ ID NO: |
|---|---|---|---|
| IFI16 | Not Secreted | 173 | 580 |
| IFI3 5 | Not Secreted | 174 | 581 |
| IFI44 | Not Secreted | 175 | 582 |
| IFI44L | Not Secreted | 176 | 583 |
| IGKC | Not Secreted | 947 | 898-942 |
| IGKV1-8 | Not Secreted | 946 | 941 |
| IGKV3-11 | secreted | 949 | 944 |
| IKZF3 | Not Secreted | 177-182 | 584-589 |
| INTS3 | Not Secreted | 183 | 590 |
| INTS7 | Not Secreted | 184 | 591 |
| INTS9 | Not Secreted | 185-186 | 592-593 |
| IPCEF1 | Not Secreted | 187-189 | 594-596 |
| IRF9 | Not Secreted | 190 | 597 |
| ITGA4 | secreted | 191 | 598 |
| ITGB7 | secreted | 192 | 599 |
| IVD | Not Secreted | 193-194 | 600-601 |
| JAK2 | Not Secreted | 195 | 602 |
| KEAP1 | Not Secreted | 196-197 | 603-604 |
| KLRAP1 | | 851 | #N/A |
| KLRG1 | Not Secreted | 198 | 605 |
| KNTC1 | Not Secreted | 199 | 606 |
| LBR | Not Secreted | 200-201 | 607-608 |
| LINC00667 | | 852 | #N/A |
| LINC00837 | | 853-855 | #N/A |
| LRRC42 | Not Secreted | 202 | 609 |
| LSM2 | Not Secreted | 203 | 610 |
| LYST | Not Secreted | 204 | 611 |
| M6PR | Not Secreted | 205 | 612 |
| MAF | Not Secreted | 206-207 | 613-614 |
| MAGEH1 | Not Secreted | 208 | 615 |
| MAN1A1 | secreted | 209 | 616 |
| MANEA | Not Secreted | 210 | 617 |
| MAVS | Not Secreted | 211 | 618 |
| MDM2 | Not Secreted | 212-215 | 619-622 |
| MELK | Not Secreted | 216 | 623 |
| METTL18 | Not Secreted | 823 | 901 |
| METTL2A | Not Secreted | 217 | 624 |
| METTL2B | Not Secreted | 218 | 625 |

(continued)

| Gene/Marker | Secreted/not secreted? | Polynucleotide SEQ ID NO: | Polypeptide SEQ ID NO: |
|---|---|---|---|
| MRE11A | Not Secreted | 219-220 | 626-627 |
| MRPL18 | secreted | 221 | 628 |
| MRPS27 | Not Secreted | 222 | 629 |
| MRPS34 | Not Secreted | 223 | 630 |
| MSH2 | Not Secreted | 224 | 631 |
| MSH3 | Not Secreted | 225 | 632 |
| MTF2 | Not Secreted | 226-229 | 633-636 |
| MTHFD2 | secreted | 230 | 637 |
| MYL6 | secreted | 231-232 | 638-639 |
| MYO5A | secreted | 233-234 | 640-641 |
| NCKAP1L | secreted | 235 | 642 |
| NDC80 | Not Secreted | 236 | 643 |
| NEAT1 | | 856-857 | #N/A |
| NEDD4 | secreted | 237-238 | 644-645 |
| NFAT5 | Not Secreted | 239-243 | 646-650 |
| NMI | Not Secreted | 244 | 651 |
| NUPL2 | Not Secreted | 245 | 652 |
| OAZ3 | Not Secreted | 246-247 | 653-654 |
| OPTN | Not Secreted | 248-251 | 655-658 |
| OSGEPL1 | Not Secreted | 252 | 659 |
| PDCD10 | secreted | 253-255 | 660-662 |
| PDIA3 | secreted | 256 | 663 |
| PDIA4 | Not Secreted | 257 | 664 |
| PDIA6 | secreted | 258 | 665 |
| PECR | Not Secreted | 259 | 666 |
| PHKB | Not Secreted | 260-261 | 667-668 |
| PLCG2 | secreted | 262 | 669 |
| PLEK | secreted | 263 | 670 |
| PLEKHG6 | Not Secreted | 264-266 | 671-673 |
| PPIA | secreted | 267 | 674 |
| PPIAP 11 | | 880 | #N/A |
| PPIAP22 | | 881 | #N/A |
| PPIAP29 | | 882 | #N/A |
| PPIAP31 | | 883 | #N/A |
| PRF1 | Not Secreted | 268-269 | 675-676 |
| PRKACB | secreted | 270-272 | 677-679 |
| PRKCB | secreted | 273-274 | 680-681 |
| PRPS2 | secreted | 275-276 | 682-683 |

(continued)

| Gene/Marker | Secreted/not secreted? | Polynucleotide SEQ ID NO: | Polypeptide SEQ ID NO: |
|---|---|---|---|
| PRSS23 | secreted | 277 | 684 |
| PSMA6 | secreted | 278 | 685 |
| PSMA6P1 | | 884 | #N/A |
| PSMB10 | Not Secreted | 279 | 686 |
| PSMB9 | secreted | 280 | 687 |
| PSME1 | secreted | 281-282 | 688-689 |
| PSME2 | secreted | 283 | 690 |
| PSME2P2 | | 858 | #N/A |
| PSMG1 | Not Secreted | 284-285 | 691-692 |
| PTPRJ | secreted | 286-287 | 693-694 |
| PTPRN2 | Not Secreted | 288-290 | 695-697 |
| PZP | secreted | 291 | 698 |
| RAB11A | secreted | 292 | 699 |
| RAB27A | secreted | 293-296 | 700-703 |
| RAB29 | secreted | 845-848 | 923-926 |
| RAP1GDS1 | secreted | 297-302 | 704-709 |
| RAP2A | secreted | 303 | 710 |
| RBL1 | Not Secreted | 304-305 | 711-712 |
| RBM28 | Not Secreted | 306-307 | 713-714 |
| RCAN1 | Not Secreted | 308-310 | 715-717 |
| RCN1 | Not Secreted | 311 | 718 |
| RGP1 | Not Secreted | 312 | 719 |
| RSAD2 | Not Secreted | 313 | 720 |
| RUVBL1 | secreted | 314 | 721 |
| SAC3D1 | Not Secreted | 315 | 722 |
| SAMD9 | Not Secreted | 316 | 723 |
| SBSPON | secreted | 844 | 922 |
| SCP2 | secreted | 317-321 | 724-728 |
| SF3B4 | Not Secreted | 322 | 729 |
| SH2D1A | Not Secreted | 323-324 | 730-731 |
| SLAMF7 | Not Secreted | 325 | 732 |
| SLC27A3 | Not Secreted | 326 | 733 |
| SMC4 | Not Secreted | 327-328 | 734-735 |
| SMC6 | Not Secreted | 329-330 | 736-737 |
| SNORD14C | | 859 | #N/A |
| SNTB2 | secreted | 331 | 738 |
| SP140 | Not Secreted | 332-333 | 739-740 |
| SPATS2L | Not Secreted | 334-337 | 741-744 |

(continued)

| Gene/Marker | Secreted/not secreted? | Polynucleotide SEQ ID NO: | Polypeptide SEQ ID NO: |
|---|---|---|---|
| SPHAR | Not Secreted | 338 | 745 |
| SQRDL | Not Secreted | 339 | 746 |
| STAT1 | Not Secreted | 340-341 | 747-748 |
| STMN1 | secreted | 342-345 | 749-752 |
| STOM | secreted | 346-347 | 753-754 |
| SUMF2 | Not Secreted | 348-352 | 755-759 |
| SUPT4H1 | Not Secreted | 353 | 760 |
| TAOK3 | Not Secreted | 354 | 761 |
| TAP1 | Not Secreted | 355 | 762 |
| TARS | secreted | 356 | 763 |
| TBK1 | Not Secreted | 357 | 764 |
| TFDP1 | Not Secreted | 358 | 765 |
| TGFBR3 | secreted | 359 | 766 |
| TMEM140 | Not Secreted | 360 | 767 |
| TMEM187 | Not Secreted | 361 | 768 |
| TMEM189-UBE2V1 | Not Secreted | 362-368 | 769-775 |
| TMEM230 | Not Secreted | 825-828 | 903-906 |
| TMX1 | Not Secreted | 369 | 776 |
| TOX | Not Secreted | 370 | 777 |
| TRAC | Not Secreted | 876 | #N/A |
| TRAF3IP3 | Not Secreted | 371 | 778 |
| TRAFD1 | Not Secreted | 372-373 | 779-780 |
| TRAV8-3 | Not Secreted | 875 | #N/A |
| TRGC1 | Not Secreted | 814-815 | 889-890 |
| TRGC2 | Not Secreted | 888 | 899-940 |
| TRGV3 | Not Secreted | 948 | 943 |
| TRGV5 | Not Secreted | 874 | 900 |
| TRGV5P | | 885 | #N/A |
| TRGV7 | | 886 | #N/A |
| TRIM22 | Not Secreted | 374 | 781 |
| TRIP 11 | Not Secreted | 375 | 782 |
| TTC38 | secreted | 376 | 783 |
| UBE2L6 | Not Secreted | 377-378 | 784-785 |
| UBE2V1 | secreted | 816-822 | 891-897 |
| UCK2 | Not Secreted | 379 | 786 |
| UNG | Not Secreted | 380-381 | 787-788 |
| USP18 | Not Secreted | 382 | 789 |
| USP21 | Not Secreted | 383-384 | 790-791 |

(continued)

| Gene/Marker | Secreted/not secreted? | Polynucleotide SEQ ID NO: | Polypeptide SEQ ID NO: |
|---|---|---|---|
| VMP1 | Not Secreted | 832 | 910 |
| VOPP1 | Not Secreted | 385 | 792 |
| VPS8 | Not Secreted | 386-387 | 793-794 |
| VRK2 | Not Secreted | 388-392 | 795-799 |
| WDR70 | Not Secreted | 393 | 800 |
| XAF1 | Not Secreted | 394-395 | 801-802 |
| XPNPEP2 | secreted | 396 | 803 |
| XRCC4 | Not Secreted | 397-399 | 804-806 |
| XRCC5 | Not Secreted | 400 | 807 |
| YWHAQ | secreted | 401 | 808 |
| ZCCHC6 | Not Secreted | 402 | 809 |
| ZMYM6NB | Not Secreted | 824 | 902 |
| ZNF16 | Not Secreted | 403-404 | 810-811 |
| GenelMarker | Secreted/not secreted? | Polynucleotide SEQ ID NO: | Polypeptide SEQ ID NO: |
| ZNF224 | Not Secreted | 405 | 812 |
| ZNF695 | Not Secreted | 406 | 813 |
| PD-1 | | 887 | 945 |
| CD57 | | 860-861 | 927-928 |
| CTLA-4 | | 862-863 | 929-930 |
| LAG-3 | | 864 | 931 |
| Tim-3 | | 865 | 932 |
| CD126 | | 866-868 | 933-935 |
| CD28 | | 869-871 | 936-938 |
| CD127 | | 872-873 | 939 |

Table 9. Provided are the gene names/markers of the T-cell senescence genetic-signature which were upregulated in senescent T-cells, along with the sequence identifiers of the genomic/mRNA/protein sequences related to these genes. It should be noted that identification of the level of T-cell senescence can be made on the genomic, RNA and/or the protein level. #N/A = sequence not available.

## EXAMPLE 2

### THE INCREASE IN T-CELL SENESCENCE DURING THE COURSE OF SEPSIS PREDCTES A MORE SEVERE CLINICAL OUTCOME OF SEPSIS

### Experimental results

Interestingly, the present inventors identified a significant correlation of severity of sepsis outcome (according to the MODS Marshal scoring) and baseline senescence enrichment, measured less than one day following trauma, establishing a link between initial immune-state and clinical outcome of sepsis (Figure ID).

*EXAMPLE 3*

***CMV SEROLOGY IS CORRELATED WITH A MORE SEVERE SEPSIS CLINICAL OUTCOME***

***Experimental results***

To unravel the clinical risk-factors to increased baseline senescence, the present inventors calculated the enrichment of the T-cell senescence genetic signature on the Framingham cohort, composed of more than 600 old individuals. In accordance to the published literature, CMV serology was the most predictive feature for increased enrichment of the senescence signature (Figure 2A). To verify the relation between baseline senescence and outcome in sepsis, the present inventors collected clinical data of 120 septic patients along with their CMV serology. The present inventors found that CMV sero-positive patients were hospitalized in the ICU for significantly prolonged time-periods compared to CMV sero-negative patients (p value <0.02), establishing the clinical impact of CMV serology on outcome in sepsis (Figure 2B).

*EXAMPLE 4*

***CYTOF ANALYSIS REVEALED GRADUAL FUNCTIONAL CHANGES IN T-CELLS DURING THE COURSE OF SEPSIS***

***Experimental results***

For further validation of the present results, the present inventors performed high-resolution profiling of the immune response to sepsis by CyTOF. CyTOF is a recently introduced technology that measures the abundance of up to 40 metal isotope labels simultaneously on single cells using mass spectroscopy. To date, changes in the immune compartment in sepsis were monitored by FACS, enabling detection of a limited number of molecules on single cells. Using CyTOF, the present inventors monitored at high resolution the peripheral-blood dynamics of septic patients, including abundances of cell subsets and functional signaling responses to IL-6 stimulation. The present inventors identified substantial shifts in the abundances of almost every cell subset during sepsis (Figure 3A).

To unravel the dynamics of senescent T cells in sepsis, CD8+T cells from the septic patients (detailed above) were clustered based on differentiation related markers, and manually annotated the clusters as naive (CCR7+ CD45RA+), memory (CCR7-CD45RA-) and senescent cells (CCR7-CD45RA+CD57+). The abundance change rate for each cluster was defined as the ratio of abundances change and time difference:

$$V_{Cluster} = \frac{abun_{cluster}(t_2) - abun_{cluster}(t_1)}{t_2 - t_1}$$

Senescent CD8+ T-cell subsets responded poorly to IL6 stimulation reflecting a functional defect of senescent T-cells in sepsis (Figure 3C). These results indicate the capability of CyTOF to detect phenotypic and functional alterations of numerous immune cells during sepsis.

The clusters' rate was evaluated at two time-points: baseline (between day 1 and the second time-points) and late phase (between the second and the third time-points). At baseline, the rates of the memory clusters were mostly negative, indicating decreasing abundances, whereas the rates of terminally-differentiated (senescent cells) clusters were mostly positive, indicating increasing abundances. In contrast, during the later phase the rates of the memory clusters increased, while the rates of the terminally-differentiated clusters decreased (Figure 1C). This result suggests highly initial effector phase that is followed by memory establishment in sepsis.

*EXAMPLE 5*

***SENEECENT CELLS ARE HIGHLY EFFECTOR SECRETING MAINLY TNFa AND IFN$\gamma$***

To achieve TCR stimulation, peripheral blood mononuclear cells were incubated with beads bearing agonistic antibodies against CD3 and CD28 to activate T cells for 18 hours. Staining for both extracellular markers for cells' classification and intra-cellular cytokines was made. All samples were profiled using CyTOF. The effector CD8+T cells (CCR7-CD8+ Tcells) were gated and further divided into 2 populations based on the expression of CD57 (CD57+Effector and CD57-Effector cells). Then the frequency of cells was calculated from each populations that were positive for the cytokines: TNFα, IFNγ and IL2. To assess polyfunctionality, the proportion of cells from each population that secret a combination of two cytokines together (double positive) was calculated. Compared to CD57 negative population, the CD57 positive population exhibits higher proportion of cells secreting IFNγ and TNFα, both are well established proinflammatory cytokines. Furthermore, CD57 positive population was more polyfunctional, with a high frequency of cells secreting concomitantly IFNg and TNFa compared to CD57negative population (Figure 4). This suggests that CD57+CD8+T cells, so called: senescent cells, exhibit an inflammatory cytokines secretion profile, therefore are a highly inflammatory cell population.

## EXAMPLE 6

## TREATMENT OF SEPTIC CELLS WITH ANTI-PD-1-ANTIBODIES RESULTED IN DOWNREGUALTION OF PD-1 AND INDUCTION OF THE CO-STIMULATORY RECEPTOR CD28

### Experimental results

Since T-cell exhaustion plays a key role in suppression of the immune response in sepsis, the present inventors aimed to reverse exhaustion *in-vitro* by targeting the inhibitory receptor PD-1. The prominent inhibitory effect of PD-1 expression on T-cell activation influence clinical outcome in sepsis by increasing mortality and nosocomial infections. PD-1 blocking antibodies are currently widely in use for boosting the immune response against the tumor in melanoma, yet there is no comprehensive study which examines their effect in sepsis. High-resolution profiling of septic peripheral blood samples that have been treated *in-vitro* with anti-PD-1 revealed a down-regulation of PD-1 by T-cells as well as significant induction of CD28, a co-stimulatory receptor essential for T-cells activation (Figures 5A-D). These results suggest that anti-PD-1 treatment might be useful to reinvigorate the immune-response in sepsis.

## Claims

1.   An ex-vivo method of determining prognosis of a subject diagnosed with sepsis, comprising determining a baseline level of T-cell senescence in a CD3+ T-cell sample of the subject up to 24 hours following diagnosis of said sepsis, as determined by at least two positive SIRS criteria, said level being indicative of the sepsis prognosis, wherein said T cell-senescence level is determined by the signature $TIM3^{low}$; $CD45RA^{high}$; $CD45RO^{low}$; $CD152^{low}$; $PD1^{low}$, thereby determining the prognosis of the subject, wherein if the T-cell senescence level is higher than a predetermined threshold then the subject is likely to have a poor prognosis as compared to a control subject diagnosed with sepsis and whose T-cell senescence level is lower than a predetermined threshold.

2.   The method of claim 1, wherein said T-cell senescence is determined in CD8 and/or CD4 T-cells.

3.   The method of claim 1, further comprising determining in a blood sample of the subject presence of CMV active or latent infection, wherein said presence of said CMV infection indicates that the subject is likely to have a poor prognosis as compared to a control subject diagnosed with sepsis and who is not infected with CMV.

4.   The method of any one of claims 1-3, wherein said determining is performed using an RNA detection method or a protein detection method.

## Patentansprüche

1.   Ex-vivo-Verfahren zur Bestimmung der Prognose eines Subjekts, bei dem eine Sepsis diagnostiziert wurde, umfassend das Bestimmen eines Basisniveaus der T-Zell-Seneszenz in einer CD3+-T-Zell-Probe des Subjekts bis zu 24 Stunden nach der Diagnose der Sepsis anhand der Bestimmung von mindestens zwei positiven SIRS-Kriterien, wobei das Niveau die Sepsis-Prognose anzeigt, wobei das Niveau der T-Zell-Seneszenz durch die Signatur $TIM3^{low}$; $CD45RA^{high}$; $CD45RO^{low}$; $CD152^{low}$; $PD1^{low}$ bestimmt wird, wodurch die Prognose des Subjekts bestimmt wird, wobei, wenn das Niveau der T-Zell-Seneszenz höher als ein vorbestimmter Schwellenwert ist, das Subjekt wahrscheinlich eine schlechte Prognose im Vergleich zu einem Kontrollsubjekt hat, bei dem eine Sepsis diagnostiziert wurde und dessen Niveau der T-Zell-Seneszenz niedriger als ein vorbestimmter Schwellenwert ist.

2.   Verfahren nach Anspruch 1, wobei die T-Zell-Seneszenz in CD8- und/oder CD4-T-Zellen bestimmt wird.

**3.** Verfahren nach Anspruch 1, ferner umfassend die Bestimmung des Vorhandenseins einer aktiven oder latenten CMV-Infektion in einer Blutprobe des Subjekts, wobei das Vorhandensein der CMV-Infektion anzeigt, dass das Subjekt wahrscheinlich eine schlechte Prognose im Vergleich zu einem Kontrollsubjekt hat, bei dem eine Sepsis diagnostiziert wurde und das nicht mit CMV infiziert ist.

**4.** Verfahren nach einem der Ansprüche 1 bis 3, wobei die Bestimmung unter Verwendung eines RNA-Nachweisverfahrens oder eines Protein-Nachweisverfahrens durchgeführt wird.

**Revendications**

**1.** Méthode ex vivo de détermination du pronostic d'un sujet diagnostiqué avec une septicémie, comprenant la détermination d'un niveau de base de sénescence des lymphocytes T dans un échantillon de lymphocytes T CD3+ du sujet jusqu'à 24 heures après le diagnostic de ladite septicémie, comme déterminé par au moins deux critères SIRS positifs, ledit niveau étant indicatif du pronostic de septicémie, ledit niveau de sénescence des lymphocytes T étant déterminé par la signature TIM3$^{bas}$ ; CD45RA$^{élevé}$ ; CD45RO$^{bas}$ ; CD152$^{bas}$; PD1$^{bas}$, déterminant ainsi le pronostic du sujet, dans laquelle si le niveau de sénescence des lymphocytes T est supérieur à un seuil prédéterminé, alors le sujet est susceptible d'avoir un pronostic défavorable par rapport à un sujet témoin diagnostiqué avec une septicémie et dont le niveau de sénescence des lymphocytes T est inférieur à un seuil prédéterminé.

**2.** Méthode selon la revendication 1, dans laquelle ladite sénescence des lymphocytes T est déterminée dans les lymphocytes T CD8 et/ou CD4.

**3.** Méthode selon la revendication 1, comprenant en outre la détermination, dans un échantillon de sang du sujet, de la présence d'une infection à CMV active ou latente, ladite présence de ladite infection à CMV indiquant que le sujet est susceptible d'avoir un pronostic défavorable par rapport à un sujet témoin diagnostiqué avec une septicémie et qui n'est pas infecté par le CMV.

**4.** Méthode selon l'une quelconque des revendications 1 à 3, dans laquelle ladite détermination est effectuée en utilisant une méthode de détection d'ARN ou une méthode de détection de protéines.

change in ES for infected/non infected patients, hospitalized above 8 days (Tcells)

Non-infected patients

Infected patients

FALSE

TRUE

density

diff

Change in enrichment-score

Fig. 1A

Fig. 1B

Fig. 1C

Fig. 1D

Fig. 2B

CMV sero-negative     CMV sero-positive

ICU hospitalization length

Fig. 2A

CMV sero-negative     CMV sero-positive

Enrichment of senescence signature

Fig. 3A

Fig. 3B

EP 3 403 091 B1

FIG. 3C

## Fig. 4

EP 3 403 091 B1

Fig. 5A

Fig. 5B

Fig. 5C

Fig. 5D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150284459 A
- US 20150274835 A
- US 20140199334 A
- US 20120021414
- US 6037137 A
- US 6350466 B
- US 20140044738, Langermann Solomon
- US 20100055102, Langermann Solomon
- US 20120114648, Langermann Solomon
- US 20120114649, Langermann Solomon
- US 20100151492, Ahmed Rafi
- US 20150284459, Kuchroo
- US 5189178 A
- US 5239078 A
- US 4036945 A, Goldenberg
- US 4331647 A
- US 4946778 A, Ladner
- US 4816567 A
- US 5545807 A
- US 5545806 A
- US 5569825 A
- US 5625126 A
- US 5633425 A
- US 5661016 A

- WO 9402610 A, Marasco
- WO 9503832 A, Duan
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A

**Non-patent literature cited in the description**

- **KOJIC, D et al.** Are there new approaches for diagnosis, therapy guidance and outcome prediction of sepsis?. *World J. Exp. Med.,* 2015, vol. 5 (2), 50-63
- **MARSHALL JC.** *Crit Care Med,* October 1995, vol. 23 (10), 1638-52
- **GENTILE LF et al.** Persistent inflammation and immunosuppression: a common syndrome and new horizon for surgical intensive care. *J. Trauma Acute Care Surg.,* 2012, vol. 72 (6), 1491-501
- **BOOMER JS et al.** Immunosuppression in patients who die of sepsis and multiple organ failure. *JAMA,* 2011, vol. 306 (23), 2594-605
- **HOTCHKISS et al.** *Nature Reviews Immunology,* 2013, vol. 13 (12), 862-874
- **HOTCHKISS et al.** *The Lancet Infectious Diseases,* 2011, vol. 13 (3), 260-268
- **INOUE S et al.** Persistent inflammation and T-cell exhaustion in severe sepsis in the elderly. *Crit Care,* 2014, vol. 18 (3), R130

- **CHANG K et al.** Targeting the programmed cell death 1: programmed cell death ligand 1 pathway reverses T-cell exhaustion in patients with sepsis. *Crit Care,* 04 January 2014, vol. 18 (1), R3
- **ROHIT MITTAL et al.** Getting older can be exhausting. *Crit Care,* 2014, vol. 18 (4), 465
- **POTTAYIL G. N. et al.** *Crit Care,* 2011, vol. 15 (2), R77
- **BRICE GAUDILLIÈRE et al.** *Sci Transl Med,* 2014, vol. 6, 255-131
- **RAQUEL ALMANSA et al.** Transcriptomic correlates of organ failure extent in sepsis. *JOURNAL OF INFECTION., GB,* 31 December 2014, vol. 70 (5), ISSN 0163-4453, 445-456
- Clinical relevance of the severe abnormalities of the T cell compartment in septic shock patients. **MONSERRAT JORGE et al.** CRITICAL CARE. BIOMED CENTRAL LTD, 25 February 2009, vol. 13, R26

- **CHARALAMBOS A. GOGOS et al.** Pro- versus Anti-inflammatory Cytokine Profile in Patients with Severe Sepsis: A Marker for Prognosis and Future Therapeutic Options. *JOURNAL OF INFECTIOUS DISEASES. JID, US,* 01 January 2000, vol. 181 (1
- **JENNIFER P. CHOU et al.** T Cell Replicative Senescence in Human Aging. *Current Pharmaceutical Design, United Arab Emirates,* 01 March 2013, 1680-1698, https://www.ncbi.nlm.nih.gov/pmc/articles/PMC3749774/pdf/nihms497521.pdf
- **RAUL DE PABLO et al.** Role of Circulating Lymphocytes in Patients with Sepsis. *BIOMED RESEARCH INTERNATIONAL,* 01 January 2014, vol. 2014, ISSN 2314-6133, 1-11
- **G. K. PHILIPS et al.** Therapeutic uses of anti-PD-1 and anti-PD-L1 antibodies. *INTERNATIONAL IMMUNOLOGY,* 16 October 2014, vol. 27 (1), ISSN 0953-8178, 39-46
- **DELLINGER RP et al.** Surviving sepsis campaign: international guidelines for management of severe sepsis and septic shock: 2012. *Crit Care Med,* February 2013, vol. 41 (2), 580-637
- **MARSHALL JC ; COOK DJ ; CHRISTOU NV.** Multiple organ dysfunction score: a reliable descriptor of a complex clinical outcome. *Crit Care Med.,* October 1995, vol. 23 (10), 1638-52
- **RICHARD P. HAUGLAND.** Molecular Probes: Handbook of Fluorescent Probes and Research Chemicals 1992-1994. Molecular Probes, Inc, 1994
- **HERMANSON.** Bioconjugate Techniques. Academic Press New York, 1995
- **KAY M et al.** *Biochemistry,* 1995, vol. 34, 293
- **STUBBS et al.** *Biochemistry,* 1996, vol. 35, 937
- Evaluating Receptor Stoichiometry by Fluorescence Resonance Energy Transfer. **GAKAMSKY D et al.** Receptors: A Practical Approach. Oxford University Press, 2001
- **KHATKHATAY MI. ; DESAI M.** *J Immunoassay,* 1999, vol. 20, 151-83
- **WISDOM GB.** *Methods Mol Biol,* 1994, vol. 32, 433-40
- **ISHIKAWA E et al.** *J Immunoassay,* 1983, vol. 4, 209-327
- **OELLERICH M.** *J Clin Chem Clin Biochem,* 1980, vol. 18, 197-208
- **SCHUURS AH. ; VAN WEEMEN BK.** *J Immunoassay,* 1980, vol. 1, 229-49
- **DENKBERG, G et al.** *Eur. J. Immunol.,* 2000, vol. 30, 3522-3532
- **CLOUTIER SM et al.** *Molecular Immunology,* 2000, vol. 37, 1067-1077
- **DUBEL S et al.** *J Immunol Methods,* 1995, vol. 178, 201
- **HUSTON JS et al.** *Methods in Enzymology,* 1991, vol. 203, 46
- **KIPRIYANOV SM et al.** *Hum Antibodies Hybridomas,* 1995, vol. 6, 93
- **KIPRIYANOV SM et al.** *Protein Engineering,* 1996, vol. 9, 203
- **PEARCE LA et al.** *Biochem Molec Biol Intl,* 1997, vol. 42, 1179-1188
- **VIJAY K et al.** *METHODS FOR MODULATING IMMUNE RESPONSES DURING CHRONIC IMMUNE CONDITIONS BY TARGETING IL-27 INDUCED PATHWAYS*
- Remington's Pharmaceutical Sciences. Mack Publishing Co,
- **FINGL et al.** The Pharmacological Basis of Therapeutics. 1975, 1
- **NUOVO GJ et al.** Intracellular localization of polymerase chain reaction (PCR)-amplified hepatitis C cDNA. *Am J Surg Pathol,* 1993, vol. 17, 683-90
- **KOMMINOTH P et al.** Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. *Pathol Res Pract,* 1994, vol. 190, 1017-25
- DNA Microarrays: Analyzing Genome-Wide Expression. **W. H. FREEMAN et al.** Molecular Cell Biology. 2000
- **ORLANDI D.R. et al.** *Proc. Natl. Acad. Sci. U. S. A.,* 1989, vol. 86, 3833-3837
- **WINTER G. et al.** *Nature,* 1991, vol. 349, 293-299
- **KOHLER G et al.** *Nature,* 1975, vol. 256, 495-497
- **KOZBOR D et al.** *J. Immunol. Methods,* 1985, vol. 81, 31-42
- **COTE RJ et al.** *Proc. Natl. Acad. Sci. U. S. A.,* 1983, vol. 80, 2026-2030
- **COLE SP et al.** *Mol. Cell. Biol.,* 1984, vol. 62, 109-120
- **HARLOW ; LANE.** Antibodies: A Laboratory Manual. Cold Spring Harbor Laboratory, 1988
- **PORTER, RR. ; 1959.** *Biochem. J.,* vol. 73, 119-126
- **INBAR et al.** *Proc. Natl. Acad. Sci. USA.,* 1972, vol. 69, 2659-62
- **WHITLOW ; FILPULA.** *Methods,* 1991, vol. 2, 97-105
- **BIRD et al.** *Science,* 1988, vol. 242, 423-426
- **PACK et al.** *Bio/Technology,* 1993, vol. 11, 1271-77
- **LARRICK ; FRY.** *Methods,* 1991, vol. 2, 106-10
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **RIECHMANN et al.** *Nature,* 1988, vol. 332, 323-327

- **VERHOEYEN et al.** *Science,* 1988, vol. 239, 1534-1536
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, vol. 77
- **BOERNER et al.** *J. Immunol.,* 1991, vol. 147, 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **RICHARDSON ; MARASCO.** *TIBTECH,* 1995, vol. 13
- **RICHARDSON et al.** *Proc. Natl. Acad. Sci. USA,* 1995, vol. 92, 3137-3141
- **DESHANE et al.** *Gene Ther,* 1994, vol. 1, 332-337
- **MARASCO et al.** *Human Gene Ther,* 1998, vol. 9, 1627-42
- **SHAHEEN et al.** *J. Virol.,* 1996, vol. 70, 3392-400
- **WERGE, T. M. et al.** *FEBS Letters,* 1990, vol. 274, 193-198
- **CARLSON, J.R.** *Proc. Natl. Acad. Sci. USA,* 1993, vol. 90, 7427-7428
- **BIOCCA, S et al.** *Bio/Technology,* 1994, vol. 12, 396-399
- **CHEN, S-Y et al.** *Human Gene Therapy,* 1994, vol. 5, 595-601
- **DUAN, L et al.** *Proc. Natl. Acad. Sci. USA,* 1994, vol. 91, 5075-5079
- **CHEN, S-Y et al.** *Proc. Natl. Acad. Sci. USA,* 1994, vol. 91, 5932-5936
- **BEERLI, R.R. et al.** *J. Biol. Chem.,* 1994, vol. 269, 23931-23936
- **MHASHILKAR, A.M. et al.** *EMBO J,* 1995, vol. 14, 1542-1551
- **KABAT, E. A. et al.** Sequences of Proteins of Immunological Interest. U.S. Department of Health and Human Services, 1991
- **SAMBROOK et al.** *Molecular Cloning: A laboratory Manual,* 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL, B.** *A Practical Guide to Molecular Cloning,* 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **BARBIE, D. A. et al.** *Systematic RNA interference reveals that oncogenic KRAS -driven cancers require TBK1,* November 2009, 462
- *Crit Care Med,* October 1995, vol. 23 (10), 1638-52